# EUROPEAN PATENT SPECIFICATION

(11) **EP 0 267 723 B1**
(45) Date of publication and mention of the grant of the patent: **07.02.1996**
(21) Application number: 87309688.7
(22) Date of filing: 02.11.1987
(51) Int. Cl.: C07D 501/00, A61K 31/545

(54) **New substituted cephalosporin sulfones as anti-inflammatory and anti-degenerative agents**
Substituierte Cephalosporinsulfone als anti-entzündliche und anti-degenerative Mittel
Sulfones de céphalosporine substituées comme agents anti-inflammatoires et anti-dégénératifs

(30) Priority: 12.11.1986 US 930193
(43) Date of publication of application: 18.05.1988
(73) Proprietor: MERCK & CO. INC., Rahway New Jersey 07065-0900 (US)
(72) Inventor: Doherty, James B., New Milford New Jersey 07646 (US); Finke, Paul E., Milltown New Jersey 08850 (US); Firestone, Raymond A., Westfield New Jersey 07090 (US); Hagmann, William K., Westfield New Jersey 07090 (US); Thompson, Kevan R., Westfield New Jersey 07090 (US); Shah, Shrenik K., Metuchen New Jersey 08840 (US)
(74) Representative: Cole, William Gwyn

(56) References cited:
- EP-A- 0 124 081
- EP-A- 0 207 447
- US-A- 4 623 645

## Description

### BACKGROUND OF THE INVENTION

We have found that sulfones of a group of new substituted cephalosporins are potent elastase inhibitors and therefore are useful anti-inflammatory/antidegenerative agents.

Proteases from granulocytes and macrophages have been reported to be responsible for the chronic tissue destruction mechanisms associated with inflammation, including rheumatoid arthritis and emphysema. Accordingly, specific and selective inhibitors of these proteases are candidates for potent anti-inflammatory agents useful in the treatment of inflammatory conditions resulting in connective tissue destruction, e.g. rheumatoid arthritis, emphysema, bronchial inflammation, osteoarthritis, spondylitis, lupus, psoriasis and acute respiratory distress syndrome.

The role of proteases from granulocytes, leukocytes or macrophages are related to a rapid series of events which occurs during the progression of an inflammatory condition:
(1) There is a rapid production of prostaglandins (PG) and related compounds synthesized from arachidonic acid. This PG synthesis has been shown to be inhibited by aspirin-related nonsteroidal anti-inflammatory agents including indomethacin and phenylbutazone. There is some evidence that protease inhibitors prevent PG production;
(2) There is also a change in vascular permeability which causes a leakage of fluid into the inflamed site and the resulting edema is generally used as a marker for measuring the degree of inflammation. This process has been found to be induced by the proteolytic or peptide cleaving activity of proteases, especially those contained in the granulocyte, and thereby can be inhibited by various synthetic protease inhibitors, for example, N-acyl benzisothiazolones and the respective 1,1-dioxides. Morris Zimmerman et al., J. Biol. Chem., 255, 9848 (1980); and
(3) There is an appearance and/or presence of lymphoid cells, especially macrophages and polymorphonuclear leukocytes (PMN). It has been known that a variety of proteases are released from the macrophages and PMN, further indicating that the proteases do play an important role in inflammation.

In general, proteases are an important family of enzymes within the peptide bond cleaving enzymes whose members are essential to a variety of normal biological activities, such as digestion, formation and dissolution of blood clots, the formation of active forms of hormones, the immune reaction to foreign cells and organisms, etc., and in pathological conditions such as the degradation of structural proteins at the articular cartilage/pannus junction in rheumatoid arthritis etc.

Elastase is one of the proteases. It is an enzyme capable of hydrolyzing the connective tissue component elastin, a property not contained by the bulk of the proteases present in mammals. It acts on a protein's nonterminal bonds which are adjacent to an aliphatic amino acid. Neutrophil elastase is of particular interest because it has the broadest spectrum of activity against natural connective tissue substrates. In particular, the elastase of the granulocyte is important because, as described above, granulocytes participate in acute inflammation and in acute exacerbation of chronic forms of inflammation which characterize many clinically important inflammatory diseases.

Proteases may be inactivated by inhibitors which block the active site of the enzyme by binding tightly thereto. Naturally occurring protease inhibitors form part of the control or defense mechanisms that are crucial to the well-being of an organism. Without these control mechanisms, the proteases would destroy any protein within reach. The naturally occurring enzyme inhibitors have been shown to have appropriate configurations which allow them to bind tightly to the enzyme. This configuration is part of the reason that inhibitors bind to the enzyme so tightly (see Stroud, "A Family of Protein-Cutting Proteins" Sci. Am. July 1974, pp. 74-88). For example, one of the natural inhibitors, α₁-Antitrypsin, is a glycoprotein contained in human serum that has a wide inhibitory spectrum covering, among other enzymes, elastase both from the pancreas and the PMN. This inhibitor is hydrolyzed by the proteases to form a stable acyl enzyme in which the active site is no longer available. Marked reduction in serum α₁-antitrypsin, either genetic or due to oxidants, has been associated with pulmonary emphysema which is a disease characterized by a progressive loss of lung elasticity and resulting respiratory difficulty. It has been reported that this loss of lung elasticity is caused by the progressive, uncontrolled proteolysis or destruction of the structure of lung tissue by proteases such as elastase released from leukocytes. J. C. Powers, TIBS, 211 (1976).

Rheumatoid arthritis is characterized by a progressive destruction of articular cartilage both on the free surface bordering the joint space and at the erosion front built up by synovial tissue toward the cartilage. This destruction process, in turn, is attributed to the protein-cutting enzyme elastase which is a neutral protease present in human granulocytes. This conclusion has been supported by the following observations:
(1) Recent histochemical investigations showed the accumulation of granulocytes at the cartilage/pannus junction in rheumatoid arthritis; and
(2) a recent investigation of mechanical behavior of cartilage in response to attack by purified elastase demonstrated the direct participation of granulocyte enzymes, especially elastase, in rheumatoid cartilage destruction. H. Menninger et al., in Biological Functions of Proteinases, H. Holzer and H. Tschesche, eds. Springer-Verlag, Berlin, Heidelburg, New York, pp. 196-206, 1979.

U.S. patent No. 4 623 645 describes certain substituted cephalosporin sulfoxides. EP 207 447 discloses certain cephalosporins having protective groups attached via a -C(O)NH- linking group.

European patent publication No. 124 081 describes a class of substituted cephalosporin sulfones which exhibit antiinflammatory and antidegenerative activity. This document does not disclose the selected active heterocyclic substituted compounds of the present invention.

Accordingly, an object of this invention is to discover new protease inhibitors, especially elastase inhibitors, useful for controlling tissue damage and various inflammatory or degenerative conditions mediated by proteases particularly elastase.

Another object of the present invention is to provide pharmaceutical compositions for administering the active substituted cephalosporin sulfones as protease inhibitors.

Still a further object of this invention is to provide a method of controlling inflammatory conditions by administering a sufficient amount of one or more of the active, substituted cephalosporin sulfones in a mammalian species in need of such treatment.

This invention relates to new cephalosporin sulfones as potent elastase inhibitors useful in the prevention, control and treatment of inflammatory conditions especially arthritis and emphysema.

Some of the cephalosporin free acids are known antibiotics which have been described in U.S. Patent No. 4,297,488 issued October 27, 1981.

The structural formula of the cephalosporin sulfones of the present invention are represented as follows: 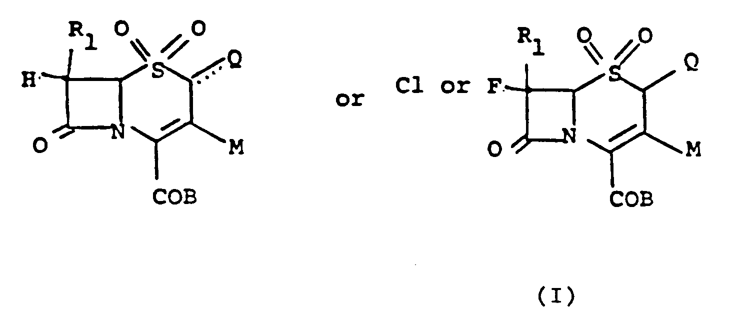
wherein M is:
(1) trifluoromethyl;
(2) chloro or fluoro;
(3) -COOH;
(4) -CHO; or
(5) -CH₂A wherein A represents
   (a) hydrogen;
   (b) halo;
   (c) ZR₅ wherein Z is oxygen and R₅ is hydrogen or straight or branched C₁₋₃ alkyl,
   (d) acyloxy wherein acyl is C₂₋₆ alkanoyl, succinoyl, or carbamoyl or thiocarbamoyl and N,-alkyl or N,N-dialkyl derivatives thereof in which alkyl groups contain 1-10 carbon atoms and may be further substituted by amino or carboxyl;
   (f) acylthio wherein the acyl group is as defined in (d);
   (g) 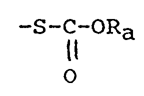 wherein Rₐ represents C₁₋₆ alkyl, phenyl or CH₂COOH;
   (h) amino, acetamido, carbamoylamino, N,N-dimethyl amino, N-(2-chloroethyl)amino, 5-cyanotriazolyl, 4-methoxy carbonyl triazol-1-yl;
   (i) Rₐ-SO- where Rₐ is C₁₋₁₆ alkyl or C₆₋₁₀ aryl
   (j) Rₐ-SO₂- where Rₐ is defined in (i) ;
   (k) a substituent selected from the group consisting of 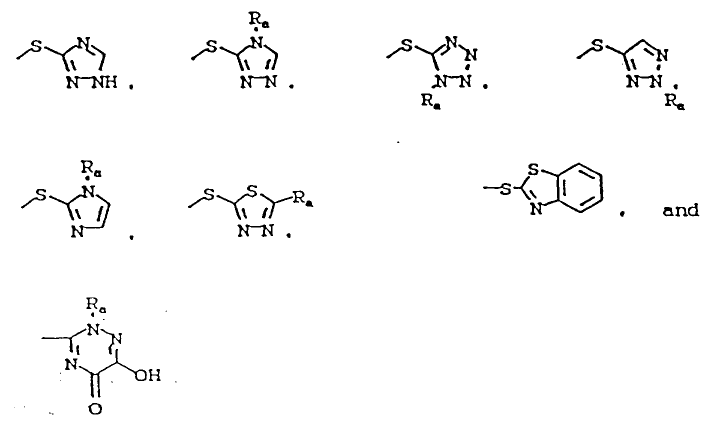 where Rₐ is C₁₋₁₆alkyl, C₆₋₁₀ aryl or CH₂CO₂H
(6) -CH=CH-Rₐ wherein Rₐ is as defined in (i);
R₁ is
(a) hydrogen;
(b) hydroxy;
(c) mercapto;
(d) OR¹₁ wherein R¹₁ is a hydrocarbyl group selected from a group consisting of straight or branched chain C₁₋₆ alkyl, C₃₋₆ alkenyl, C₃₋₆ alkynyl, phenyl and benzyl, these hydrocarbyl groups may be unsubstituted or substituted with one or more radicals which is hydroxy, halo, nitro, amino or carboxy;
(e) -SR¹₁ wherein R¹₁ is as previously defined;
(f) a hydrocarbyl group selected from a group consisting of
   (1) straight or branched C₁₋₆ alkyl;
   (2) straight or branched C₂₋₆ alkenyl;
   (3) straight or branched C₂₋₆ alkynyl;
   (4) benzyl or phenethyl;
   (5) cyclopropyl or cyclopentyl;
   (6) phenyl;
   (7) -CF₃;
   the above groups (1) to (6) can be unsubstituted or substituted with one or more radicals selected from a group consisting of halo, hydroxy, amino, nitro, sulfonyl, sylfamoyl, acyloxy, carbamoyloxy, carboxy or carboxamido;
(g) cyano;
(h) 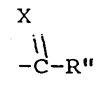 wherein X is oxygen or sulfur and R'' is
   (1) hydrogen;
   (2) hydroxy;
   (3) mercapto;
   (4) amine;
   (5) N-alkyl or N,N-dialkylamine;
   (6) C₁₋₆ alkyl;
   (7) benzyloxy;
   (8) phenoxy;
   (9) C₁₋₆ alkylthio; or
   (10) phenylthio;
(i) halo;
(j) 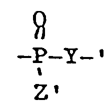 where Y' and Z' independently are OH, or -OC₁₋₆ alkyl;
(k) 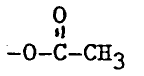
(l) -SO₂NH₂;
(m) -SO₂R₂ where R₂ is C₁₋₆ alkyl, halo C₁₋₆ alkyl, phenyl, or benzyl;
(n) -SO₂NR₃R₄ where R₃ and R₄ independently represent H or C₁₋₆ alkyl;
B is a heterocyclic group selected from-the group comprising 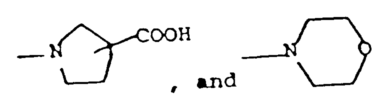
Q is
(1) hydrogen;
(2) C₁₋₆ alkyl;
(3) halo C₁₋₆ alkyl;
(4) hydroxy C₁₋₆ alkyl;
(5) methylene, C₁₋₆ alkylmethylene, phenyl methylene, phenylthiomethylene, phenylsulfinylmethylene or phenylsulfonyl methylene;
(6) C₁₋₆ alkoxy C₁₋₆ alkyl;
(7) benzyl;
(8) phenylthio C₁₋₆ alkyl, phenylsulfinyl C₁₋₆ alkyl or phenylsulfonyl C₁₋₆ alkyl;
(9) phenoxy C₁₋₆ alkyl; or
(10) phenylamino C₁₋₆ alkyl.

A favoured compound of this invention is 3-acetyloxymethyl-7α-methoxy-8-oxo-5-thia-1-azabicyclo[4·2·0·]oct-2-ene-2-(2-(S)-carboxypyrrolidinecarboxamide)-5,5-dioxide.

Other compounds of the invention are represented by the formula: 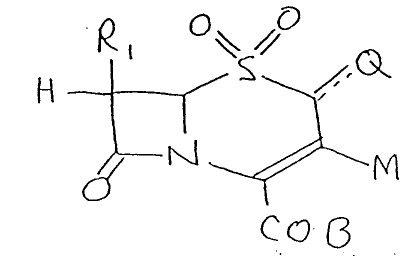
wherein M is:
(1) trifluoromethyl;
(2) chloro or fluoro; or
(3) -CH₂A wherein A represents:
   (a) alkanoyloxy of 2-6 carbon atoms
   (b) alkoxy of 1-3 carbon atoms
   (c) halo
   (d) hydrogen
   (e) hydroxy
   (f) mercapto or mercapto substituted by an aryl group of 2-6 carbon atoms
   (g) carbamoyloxy
   (h) -SO-CH₃ or - SO-C₆H₅
   (i) -SO₂-CH₃ or -SO₂-C₆H₅
R₁ is:
(1) C₁₋₆ alkyl
(2) hydroxy
(3) OR: where R¹ is
   (a) C₁₋₆ alkyl, C₃₋₆ alkenyl or C₃₋₆ alkynyl
   (b) -C₆H₅
   (c) CH₂CH₂C₆H₅ or
   (d) -CO-R¹¹ where R¹¹ is hydrogen, C₁₋₆ alkyl, phenyl, benzyl or C₁₋₆ alkylamino
(4) fluoro or chloro
B is as defined in Claim 1 and
Q is
(1) hydrogen;
(2) methyl, ethyl or i- or n- propyl;
(3) methylene or
(4) phenylthiomethyl or phenyl sulfonylmethyl.

In favoured compounds as defined above Q is hydrogen.

In favoured compounds as defined above R₁ is methoxy.

In favoured compounds as defined above M is CH₂A wherein A is alkanoyloxy.

Apt compounds of the invention are represented by the formula: 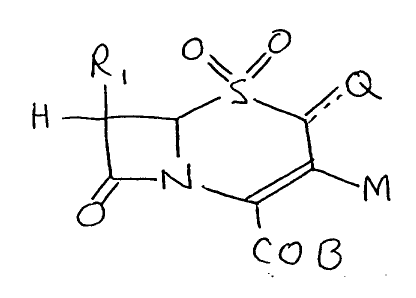
wherein M is:
(1) trifluoromethyl;
(2) chloro or fluoro; or
(3) -CH₂A wherein A represents:
   (a) alkanoyloxy of 2-6 carbon atoms
   (b) alkoxy of 1-6 carbon atoms
   (c) halo
   (d) hydrogen
   (e) hydroxy
   (f) mercapto or mercapto substituted by an aryl group of 2-6 carbon atoms
   (g) carbamoyloxy
   (h) -SO-CH₃ or - SO-C₆H₅
   (i) -SO₂-CH₃ or -SO₂-C₆H₅
R₁ is:
(1) C₁₋₆ alkyl
(2) hydroxy
(3) OR: where R¹ is
   (a) C₁₋₆ alkyl, C₃₋₆ alkenyl or C₃₋₆ alkynyl
   (b) -C₆H₅
   (c) CH₂CH₂C₆H₅ or
   (d) -CO-R¹¹ where R¹¹ is hydrogen, C₁₋₆ alkyl, phenyl, benzyl or C₁₋₆ alkylamino
(4) fluoro or chloro
B is as defined in Claim 1 and
Q is
(1) hydrogen;
(2) methyl, ethyl or i- or n- propyl;
(3) methylene or
(4) phenylthiomethyl or phenyl sulfonylmethyl.

In favoured compounds as defined above Q is hydrogen.

In favoured compounds as defined above R₁ is methoxy.

In favoured compounds as defined above M is CH₂A wherein A is alkanoyloxy.

The present invention provides a process for the preparation of a compound of the formula (I) as herein before defined which comprises treating a compound of formula 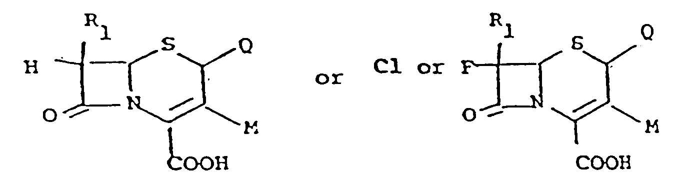
with a compound of formula HB in the presence of a catalyst.

A process for preparing the novel compounds disclosed herein comprises
(a) esterifying a compound of formula 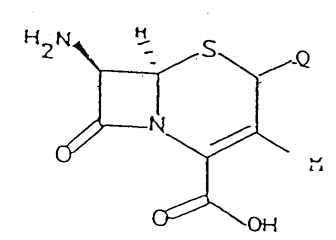 to form an ester of formula 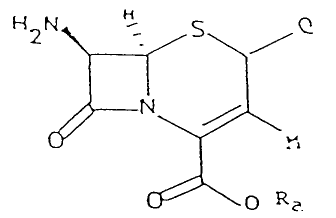 wherein Q and M are as previously defined; and Rₐ is a protecting group;
(b) diazotizing the ester of step (a) with NaNO₂ or other diazotizing reagents and converting the resulting diazo derivative with methanol and rhodium acetate dimer to form an ester of formula 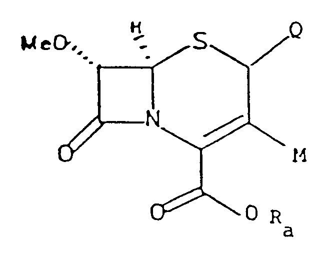 wherein Rₐ is a protecting group comprising C₁₋₆alkyl, benzyl and benzylhydryl;
(c) converting the ester formed in step (b) to a free acid of formula 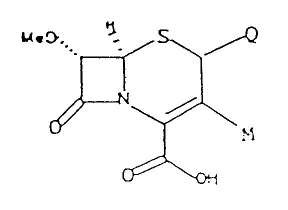
(d) reacting the free acid formed in step (c) with a heterocyclic compound of formula HB in the presence of N-hydroxysuccinimide, DCC and triethylamine to form the amide of formula 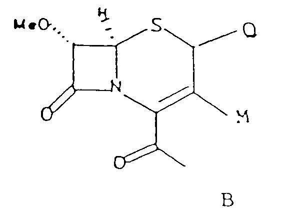 wherein B is defined previously; or alternatively esterifying the free acid of step (d) to form an ester of formula 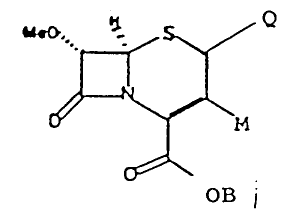 wherein B is as previously defined; and
(e) Oxidizing the product of step (d) with m-chloroperbenzoic acid or other suitable oxidation agents to form a sulfone of formula 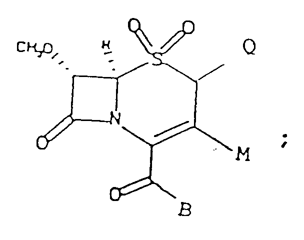 and optionally
(f) Converting the sulfone of step (e) to a compound of formula (I) by deblocking the protecting group.

Aptly the process is adapted to the preparation of a compound of formula

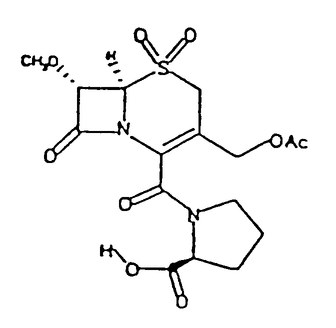

Suitable methods of preparing compounds useful in the processes of this invention are described in European Patent Application 873096887 (EP-A-0 267 723).

This invention also relates to a method of treating inflammation in patients using a compound of Formula (I), particularly an especially preferred compound as the active constituent.

It has been found that the compounds of Formula (I) have anti-inflammatory antidegeneration activity and are effective in the prevention and inhibition of edema and granuloma tissue formation as shown by the effective inhibition of the proteolytic function of human granulocyte elastase. The compound of Example 1 was found to have an ED₅₀ of 5.

Synthesis of 3-acetyloxymethyl-7α- methoxy-8-oxo-5-thia-1-azabicyclo[4,3,0]oct-2-ene-2-(2-(S)-carboxypyrrolidinecarboxamide)-5,5-dioxide may be as follows:-

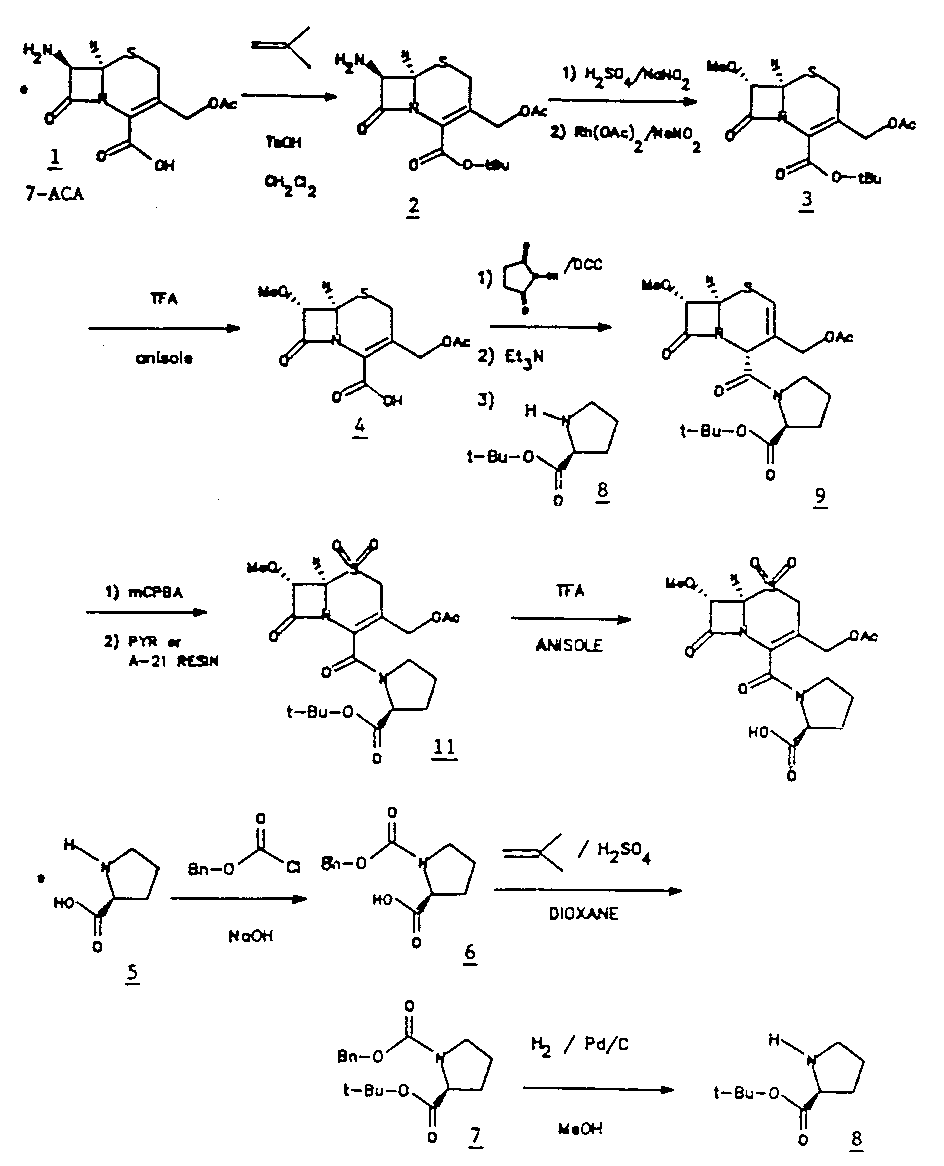

### Protocol - Enzyme Assays for the Inhibition of Human Polymorphonuclear Leukocyte Elastase Via Hydrolysis of

### N-t-Boc-alanyl-alanyl-prolylalanine-p-nitroanilide Reagents:

0.05M TES (N-tris[hydroxymethyl]methyl-2-amino-ethanesulfonic acid) Buffer, pH 7.5.

0.2 mM N-t-Boc-alanyl-alanyl-prolyl-alanine-p-nitroanilide (Boc-AAPAN).

To prepare substrate, the solid (m.w. 550) was first dissolved in 10.0 ml DMSO. Buffer at pH 7.5 was then added to a final volume of 100 ml.

Crude extract of human polymorphonuclear leukocytes (PMN) containing elastase activity.

Inhibitors (cephalosporin sulfone esters) to be tested dissolved in DMSO just before use.

### Assay Procedure:

To 1.0 ml of 0.2 mM Boc-AAPAN in a cuvette, 0.01-0.1 ml of DMSO with or without inhibitor was added. After mixing, a measurement was taken at 410 mµ to detect any spontaneous hydrolysis due to presence of test compound. 0.05 Milliliters of PMN extract was then added and the ΔOD/min at 410 mµ was measured and recorded. Beckman model 35 spectrophotometer was used.

### Results:

Results were reported as ED₅₀, i.e., effective dosage in micrograms per milliliter (µg/ml) for 50% inhibition of the enzyme activity 2 minutes after zero time. The compound of Example 1 was found to have an ED₅₀ of 5.

The elastase activity in the crude PMN extract may vary from one preparation to another. A control of each new batch is run, and the volume added in the assay procedure is adjusted according to activity.

Accordingly, the compounds of Formula (I) can be used to reduce inflammation and relieve pain in diseases such as emphysema, rheumatoid arthritis, osteoarthritis, gout, bronchial inflammation, infectious arthritis, rheumatic fever and the like.

For treatment of inflammation, fever or pain, the compounds of Formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, maize starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The said aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan mono-oleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The compounds of Formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the anti-inflammatory agents are employed.

Dosage levels of the order to 0.2 mg to 140 mg per kilogram of body weight per day are useful in the treatment of the above-indicated conditions (10 mg to 7 gms. per patient per day). For example, inflammation is effectively treated and anti-pyretic and analgesic activity manifested by the administration from about 0.5 to 50 mg of the compound per kilogram of body weight per day (25 mg to 3.5 gms per patient per day). Advantageously, from about 2 mg to about 20 mg per kilogram of body weight per daily dosage produces highly effective results (50 mg to 1 gm per patient per day).

The amount of active ingredient that may be combined with the carrier materials to produce a single dosage form will vary depending upon the host treated and the particular mode of administration. For example, a formulation intended for the oral administration of humans may contain from 5 mg to 5 gm of active agent compounded with an appropriate and convenient amount of carrier material which may vary from about 5 to about 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

### Example: Synthesis of 3-acetyloxymethyl-7α- methoxy-8-oxo-5-thia-1-azabicyclo[4,3,0]oct-2-ene-2-(2-(S)-carboxypyrrolidinecarboxamide)-5,5-dioxide.

### Step A: Preparation of t-butyl 3-acetyloxymethyl 7β-amino-8-oxo-5-thia-1-azabicyclo[4.2.0]oct-3-ene-2-carboxylate (t-butyl 7-ACA, 2)

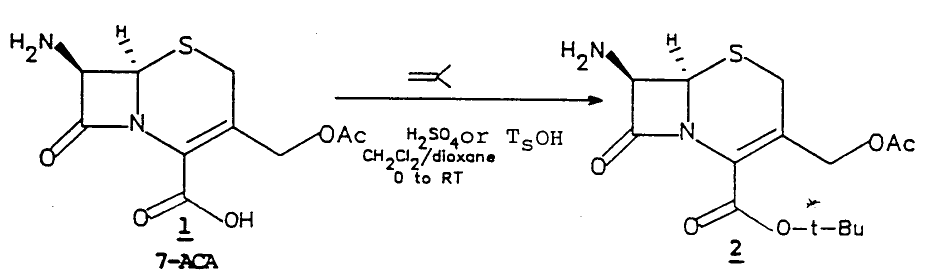

*t-Bu can be substituted by another protecting group, e.g., C₁₋₆ alkyl; benzhydryl, benzyl and the like.

A suspension of tosic acid monohydrate (500 gms, 2.6 moles) in toluene (2L) was dehydrated by refluxing under a Dean-Stark trap for 40 hrs. At this time all of the tosic acid had gone into solution and a total of 57 mL of water had been collected. The toluene was then removed in vacuo to afford a colored, solid mass.

The dehydrated tosic acid was taken up in methylene chloride (2500 mL) and transferred to a 5-L 3-necked round bottom flask which had been fitted with a mechanical stirrer, N₂ and gas inlet tubes and a dry ice condenser. Solid 7-ACA (1) (355 gms, 1.3 moles) was slowly added at a rate to achieve solution of the 7-ACA without formation of a gummy mass. The solution was then cooled to 10-15°C and isobutylene (1000 mL) was distilled into the reaction mixture over 2-1/2 hrs. Note: Rapid addition of the isobutylene causes formation of a gummy precipitate. The reaction mixture was stirred overnight at room temperature.

The reaction was slowly quenched with vigorous stirring into a 5-gallon carboy containing a solution of sodium bicarbonate (500 gms) in ice water (6L). The organic layer was separated using suction and washed with water and brine. The aqueous layers were sequentially back-extracted with two portions of methylene chloride (500 mL) and the combined layers were dried over sodium sulfate. Most of the methylene chloride was removed in vacuo until the product began to solidify. At this time cyclohexane (1L) was added and the precipitate was triturated to break up the clumps. The product was collected by filtration, washed with cyclohexane and dried overnight by pulling air through the filter cake. The yield of t-butyl 7-ACA (2) was 360 grms (80%) as an off-white solid. R_{f} (Et₂0) = 0.3 - 0.5.

### Step B: Preparation of t-butyl-3-acetyloxymethyl-7α-methoxy-8-oxo-5-thia-1-azabicyclo[4.2.0]oct-3-ene-2-carboxylate (3)

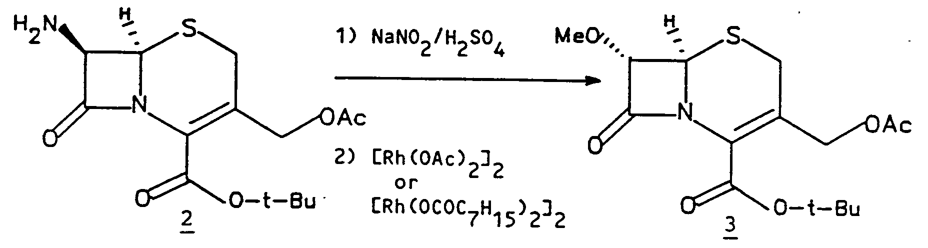

The diazotization reaction was run in two 50 gm batches and then combined for the work-up and second step.

To duplicate solutions of t-butyl 7-ACA 2 (2 x 50 gms, 0.30 moles) in methylene chloride (2 x 600 mL) was added sodium nitrite (2 x 11.4 g, 0.33 moles) dissolved in water (2 x 500 mL). The mixtures were cooled in an ice bath and to each was added 2N H₂SO₄ (2 x 114 mL, 0.450 moles) in portions over five minutes and the reactions were stirred for 45 minutes at 0°C. They were then combined and the organic layer was separated (some problems with emulsions) and washed with water and brine. The aqueous layers were back-extracted with methylene chloride and the organic layers combined, dried over sodium sulfate and filtered.

To the above methylene chloride solution was added at room temperature methanol (800 mL) and, while vigorously stirring, rhodium acetate dimer (0.64 gms). Vigorous nitrogen evolution occurred and after 1/2 hr the reaction was filtered through celite and concentrated in vacuo at low temperature. The residue was taken up in ether and washed with water and brine to remove any residual methanol. The aqueous layers were back-extracted with ether and the organic layers were combined, dried over sodium sulfate and evaporated in vacuo. The residue was purified by preparative HPLC in two portions using 15% EtOAc/hexane as eluent. The yield of 3 was 25-30 gms (25%) as a yellow oil which slowly crystallized on standing. R_{f} (25% EtOAc/hex) = 0.50. The product 3 was the first major component on TLC.

### Step C: Preparation of 3-acetyloxymethyl-7α-methoxy-8-oxo-5-thia-1-azabicyclo[4.2.0] oct-3-ene-2-carboxylic acid (4)

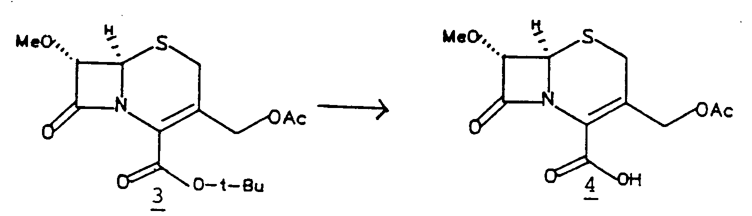

To a solution of TFA (75 mL) and anisole (5 mL) at 0° was added the t-butyl sulfide 3 (18 gms) as a solid or in a minimum amount of methylene chloride. The reaction was stirred at 0° for 1 hr or until it was judged nearly complete by TLC (Note: The reaction never seems to go to completion). The solution was then poured into a mixture of ice water (200 mL) and methylene chloride (200 mL) and the organic layer was separated and washed twice with water (100 mL). Each aqueous layer was sequentially back extracted with an additional three portions of methylene chloride (200 mL). The combined methylene chloride layers were extracted twice with water containing enough sodium bicarbonate solution to maintain the pH at 7-8 and each extract was washed with methylene chloride. The combined aqueous layers were acidified with 2N hydrochloric acid in the presence of ethyl acetate. The layers were separated and the organic layer was washed with brine. The aqueous layers were back extracted with three portions of ethyl acetate (50 mL) and these were combined, dried over sodium sulfate and evaporated. The crude product 4 was usually a dark, sticky foam and was used as obtained as soon as possible. The yield of 4 was typically 12-14 gms (80-90%).

### Step D: Preparation of N-benzoxycarbonyl-L-Proline (6)

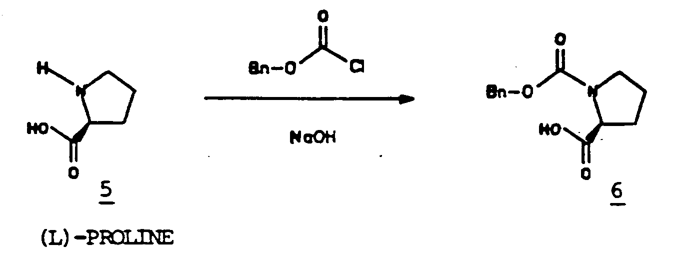

L-Proline 5 (100 gms, .87 moles) was dissolved in 2N sodium hydroxide (430 mL) and cooled in an ice bath. A further 220 mL of 4N sodium hydroxide was added simultaneously with benzyl chloroformate (155 gms, .90 moles) over 2 hrs. The reaction was then allowed to warm to room temperature overnight.

The reaction was washed twice with ether (500 mL) and the aqueous layer acidified with concentrated hydrochloric acid and extracted twice with ethyl acetate (500 mL). The combined organic layers were washed with brine and dried over sodium sulfate. Evaporation in vacuo gave 268 gms of crude 6 as a thick oil. This was used directly in the next reaction.

### Step E: Preparation of t-butyl N-benzoxycarbonyl-pyrrolidine-2-carboxylate (7)

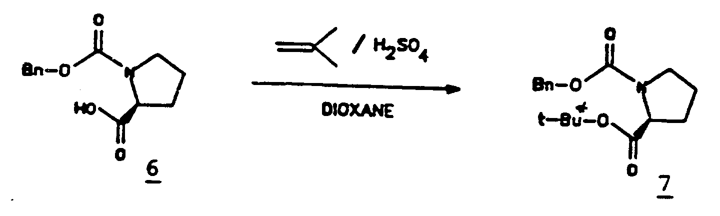

*t-Bu can be substituted by a protecting group such as C₁₋₆ alkyl, benzyl, benzhydryl and the like.

The above product 6 was taken up in dioxane (1400 mL) and placed in two 2-L pressure bottles. To each bottle was added conc. sulfuric acid (30 mL) and condensed (dry ice bath) isobutylene (350 mL).

The bottles were stoppered, fastened with wire and the reactions were stirred at room temperature overnight. The reactions became homogeneous after approximately 1 hr.

The solutions were cooled (until dioxane started to freeze), carefully vented and poured into a carboy containing a solution of sodium bicarbonate (250 gms) in ice water (4 L) and ether (2L). The layers were separated and the ether was washed with water and brine. The aqueous layers were back-extracted with ether and the ether layers were combined and dried over sodium sulfate. Evaporation in vacuo gave 185 gms of crude 7 as a clear oil. This was used directly in the next step.

### Step F: Preparation of t-butyl pyrrolidine-2-carboxylate (8)

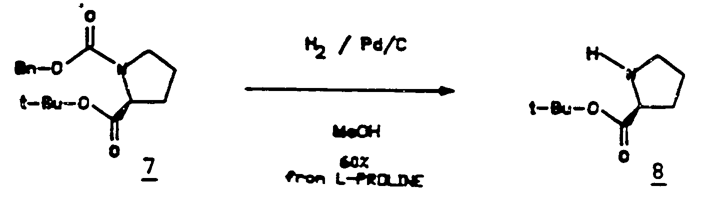

The above oil 7 was taken up in methanol (1500 mL) and hydrogenated over 10% pd/C (8 gms) for 20 hrs. (left overnight for convenience). The reaction was filtered and concentrated in vacuo without heating. The residue was distilled at 55-65° C/ 1 torr to give 85 gms (60% overall from L-proline) of t-butyl L-proline (8).

### Step G: Preparation of 3-acetyloxymethyl-7α-methoxy-8-oxo-5-thia-1-azabicyclo[4.2.0]oct-2-ene-2-(2-(s)-carboxypyrrolidinecarboxamide) (9)

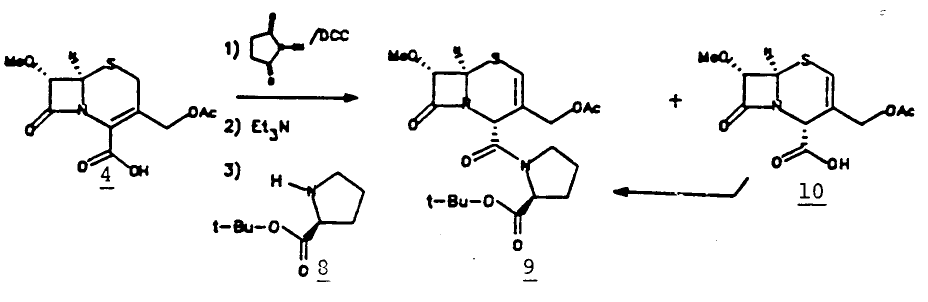

To an ice bath cooled solution of the crude acid 4 (11.5 gms, 40 mmoles) in dioxane (100 mL) are sequentially added N-hydroxysuccinimide (5.7 gms, 50 mmole) and DCC (12.4 gms, 60 mmoles). The reaction was then stirred under nitrogen at room temperature for 1/2 hr during which time a thick precipitate formed. The reaction was again cooled in an ice bath before dry triethylamine (5.5 mL, 40 mmoles) was added and the stirring was continued for another 1/2 hr. Finally, t-butyl L-proline 8 (14 gms, 80 mmole) was added all at once. After a further 2 hrs at room temperature, the reaction was diluted with ether (200 mL), filtered, and quenched into ice water (200 mL) containing 60 mL of 2N hydrochloric acid. The layers were separated and the organic layer was washed with water, sodium bicarbonate solution and brine. Each aqueous layer was back extracted with another 100 mL of ether. The organic layers were combined, dried over sodium sulfate and evaporated in vacuo. The product 9 was purified by preparative HPLC (45%) EtOAc/hexanes to give 10.5 gms (60%) of 9 as a slightly colored oil. The product 9 was usually accompanied by a small amount of Δ³ product as well as some DCC by-product.

The above sodium bicarbonate wash of was acidified in the presence of EtOAc to a pH of 1-2 layers were separated. The aqueous layer was re-extracted with another two portions of ethyl acetate and the organic layers were combined, dried over sodium sulfate and evaporated to give 0.5 - 2.0 gms of recovered Δ² acid 10. This was readily recycled similar to the above reaction to obtain 9.

### Step H: Preparation of t-butyl 3-acetoxymethyl-7α-methoxy-8-oxo-5-thia-1-azabicyclo[4.2.0.]oct-2-ene-2-(2-(s)-carboxypyrrolidine carboxamide)-5,5-dioxide (11)

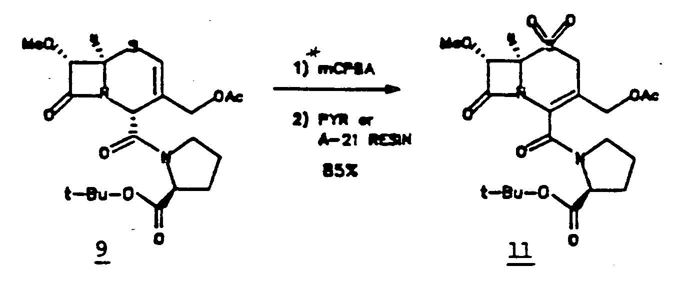

To a solution of Δ²-sulfide 9 (10.5 gms, 24 mmole) in methylene chloride (700 mL) (Note: The reaction should be kept dilute to avoid any problem with t-Bu ester loss) was added 85% m-chloroperbenzoic acid (14.7 gms, 72 mmole). The solution was stirred at room temperature overnight. The reaction was then quenched into a mixture of sodium bicarbonate and excess sodium sulfite. The layers were separated and the organic layer was washed with brine containing a few milliliters of saturated sodium sulfite solution. The aqueous layers were back extracted with methylene chloride and the organic layers were combined and dried over sodium sulfate. Pyridine (5 drops) or amberlyst A-21 resin (5-10 gms) were added and the mixture stirred for 1/2 hr in order to completely isomerize the product to Δ³. Filtration (for A-21 resin) and evaporation gave a crude residue which was purified by preparative HPLC (50% EtOAc/hexanes) to give 9.5 gms (85%) of 11 as a white foam.

### Step I: Preparation of 3-acetyloxymethyl-7α-methoxy-8-oxo-5-thia-1-azabicyclo[4.2.0]oct-2-ene-2-(2-(s)-carboxypyrrolidinecarboxamide)-5,5-dioxide

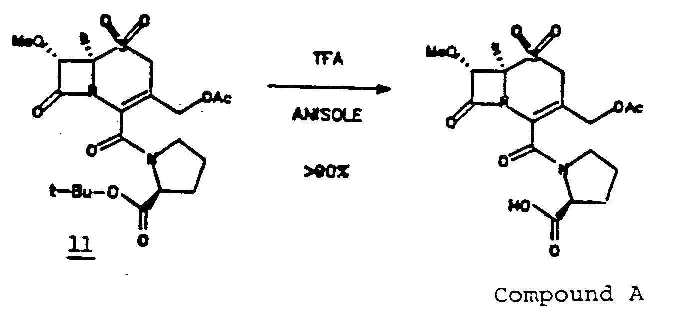

To an ice bath cooled solution of TFA (75 mL) and anisole (5 mL) was added the t-butyl sulfone 11 (8.0 gms). After stirring for 1 hr at 0°C, the reaction was evaporated in vacuo without much heating. The residue was taken up in methylene chloride and re-evaporated to remove most of the TFA. The remaining volatiles were blown off in a stream of nitrogen and the product was precipitated and triturated with ether and filtered. The product was redissolved in a minimum amount of methylene chloride and reprecipitated with ether to give 6.4 gms of off-white solid. The combined mother liquors were evaporated and the residue was flash chromatographed eluting with a solvent gradient of 60% EtOAc/hexane to 80% EtOAc/hexane and finally 1% HOAc/EtOAc to give 950 mg of pure compound A after evaporation of 50 mL of toluene to remove any residual acetic acid.

The 6.4 gms were flash chromatographed as above in 3 portions, obtaining 1.86 gms of compound A from a 2.0 gm portion. NMR (CDCl₃): δ2.08 and 2.11 (2s, 3H); 1.8-2.4 (m, 4H); 3.56 and 3.6 (2s, 3H); 3.4-4.0 (m, 2H); 3.90 (ABq, 2H); 4.4-5.0 (m, 3H); 4.90 (brs, 1H); 4.26 (brs, 1H).

## Claims (Claims for the following Contracting State(s): DE, GB, FR, IT, NL, SE, CH, LI, BE, AT, LU)

1. A compound of structural formula: 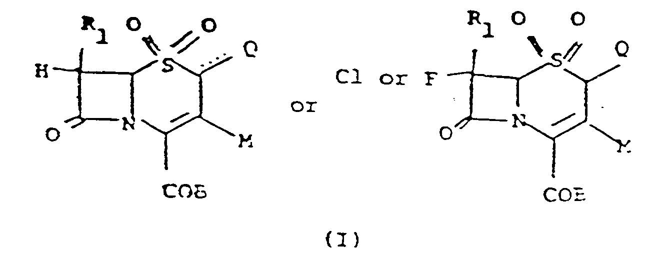 wherein M is:
(1) trifluoromethyl;
(2) chloro or fluoro;
(3) -COOH;
(4) -CHO; or
(5) -CH₂A wherein A represents
(a) hydrogen;
(b) halo;
(c) ZR₅ wherein Z is oxygen and R₅ is hydrogen or straight or branched C₁₋₃ alkyl,
(d) acyloxy wherein acyl is C₂₋₆ alkanoyl, succinoyl, or carbamoyl or thiocarbamoyl and N,-alkyl or N,N-dialkyl derivatives thereof in which alkyl groups contain 1-10 carbon atoms and may be further substituted by amino or carboxyl;
(f) acylthio wherein the acyl group is as defined in (d);
(g) 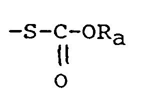 wherein Rₐ represents C₁₋₆ alkyl, phenyl or CH₂COOH;
(h) amino, acetamido, carbamoylamino, N,N-dimethyl amino, N-(2-chloroethyl)amino, 5-cyanotriazolyl, 4-methoxy carbonyl triazol-1-yl;
(i) Rₐ-SO- where Rₐ is C₁₋₁₆ alkyl or C₆₋₁₀ aryl
(j) Rₐ-SO₂- where Rₐ is defined in (i);
(k) a substituent selected from the group consisting of 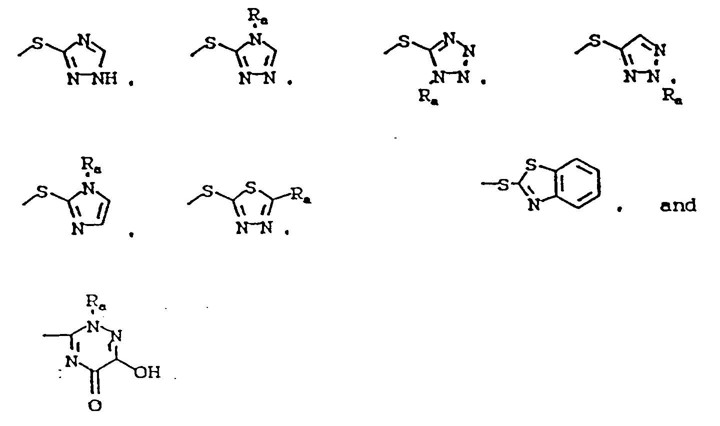 where Rₐ is C₁₋₁₆alkyl, C₆₋₁₀ aryl or CH₂CO₂H
(6) -CH=CH-Rₐ wherein Rₐ is as defined in (i);
R₁ is
(a) hydrogen;
(b) hydroxy;
(c) mercapto;
(d) OR¹₁ wherein R¹₁ is a hydrocarbyl group selected from a group consisting of straight or branched chain C₁₋₆ alkyl, C₃₋₆ alkenyl, C₃₋₆ alkynyl, phenyl and benzyl, these hydrocarbyl groups may be unsubstituted or substituted with one or more radicals which is hydroxy, halo, nitro, amino or carboxy;
(e) -SR¹₁ wherein R¹₁ is as previously defined;
(f) a hydrocarbyl group selected from a group consisting of
(1) straight or branched C₁₋₆ alkyl;
(2) straight or branched C₂₋₆ alkenyl;
(3) straight or branched C₂₋₆ alkynyl;
(4) benzyl or phenethyl;
(5) cyclopropyl or cyclopentyl;
(6) phenyl;
(7) -CF₃;
the above groups (1) to (6) can be unsubstituted or substituted with one or more radicals selected from a group consisting of halo, hydroxy, amino, nitro, sulfonyl, sylfamoyl, acyloxy, carbamoyloxy, carboxy or carboxamido;
(g) cyano;
(h) 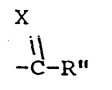 wherein X is oxygen or sulfur and R'' is
(1) hydrogen;
(2) hydroxy;
(3) mercapto;
(4) amine;
(5) N-alkyl or N,N-dialkylamine;
(6) C₁₋₆ alkyl;
(7) benzyloxy;
(8) phenoxy;
(9) C₁₋₆ alkylthio; or
(10) phenylthio;
(i) halo;
(j) 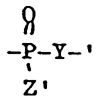 where Y' and Z' independently are OH, or -OC₁₋₆ alkyl;
(k) 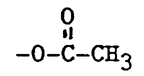
(l) -SO₂NH₂;
(m) -SO₂R₂ where R₂ is C₁₋₆ alkyl, halo C₁₋₆ alkyl, phenyl, or benzyl;
(n) -SO₂NR₃R₄ where R₃ and R₄ independently represent H or C₁₋₆ alkyl;
B is a heterocyclic group selected from-the group comprising 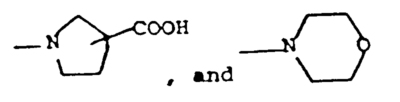 Q is
(1) hydrogen;
(2) C₁₋₆ alkyl;
(3) halo C₁₋₆ alkyl;
(4) hydroxy C₁₋₆ alkyl;
(5) methylene, C₁₋₆ alkylmethylene, phenyl methylene, phenylthiomethylene, phenylsulfinylmethylene or phenylsulfonyl methylene;
(6) C₁₋₆ alkoxy C₁₋₆ alkyl;
(7) benzyl;
(8) phenylthio C₁₋₆ alkyl, phenylsulfinyl C₁₋₆ alkyl or phenylsulfonyl C₁₋₆ alkyl;
(9) phenoxy C₁₋₆ alkyl; or
(10) phenylamino C₁₋₆ alkyl.

2. The compound of Claim 1 which is 3-acetyloxymethyl-7α-methoxy-8-oxo-5-thia-1-azabicyclo[4·2·0·]oct-2-ene-2-(2-(S)-carboxypyrrolidinecarboxamide)-5,5-dioxide.

3. A compound of the formula: 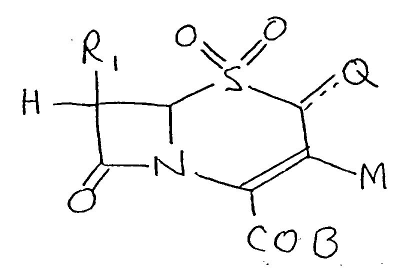 wherein M is:
(1) trifluoromethyl;
(2) chloro or fluoro; or
(3) -CH₂A wherein A represents:
(a) alkanoyloxy of 2-6 carbon atoms
(b) alkoxy of 1-3 carbon atoms
(c) halo
(d) hydrogen
(e) hydroxy
(f) mercapto or mercapto substituted by an aryl group of 2-6 carbon atoms
(g) carbamoyloxy
(h) -SO-CH₃ or - SO-C₆H₅
(i) -SO₂-CH₃ or -SO₂-C₆H₅
R₁ is:
(1) C₁₋₆ alkyl
(2) hydroxy
(3) OR: where R¹ is
(a) C₁₋₆ alkyl, C₃₋₆ alkenyl or C₃₋₆ alkynyl
(b) -C₆H₅
(c) CH₂CH₂C₆H₅ or
(d) -CO-R¹¹ where R¹¹ is hydrogen, C₁₋₆ alkyl, phenyl, benzyl or C₁₋₆ alkylamino
(4) fluoro or chloro
B is as defined in Claim 1 and
Q is
(1) hydrogen;
(2) methyl, ethyl or i- or n- propyl;
(3) methylene or
(4) phenylthiomethyl or phenyl sulfonylmethyl.

4. A compound as claimed in claim 3 wherein Q is hydrogen.

5. A compound as claimed in claim 3 or 4 wherein R₁ is methoxy.

6. A compound as claimed in any of claims 3 to 5 wherein M is CH₂A wherein A is alkanoyloxy.

7. A process for preparing a compound according to Claim 1 having structural formula (I) 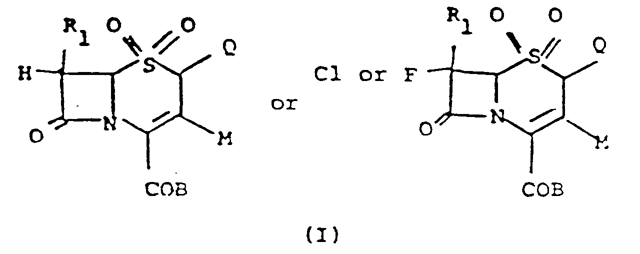 wherein M, R₁, B & Q are defined in claim 1, comprising
(a) treating a compound of formula 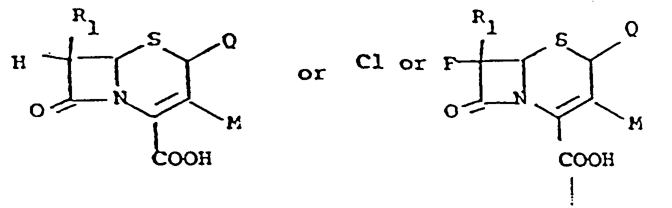 with a compound of formula HB in the presence of a catalyst; and
(b) oxidizing the product from step (a) to a compound of formula (I).

8. A process for preparing a compound of Claim 1 comprising
(a) esterifying a compound of formula 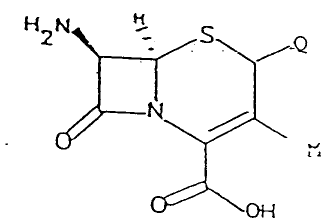 to form an ester of formula 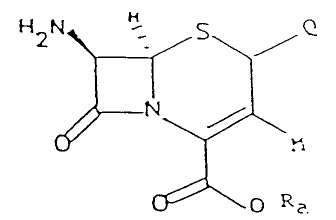 wherein Q and M are as previously defined; and Rₐ is a protecting group;
(b) diazotizing the ester of step (a) with NaNO₂ or other diazotizing reagents and converting the resulting diazo derivative with methanol and rhodium acetate dimer to form an ester of formula 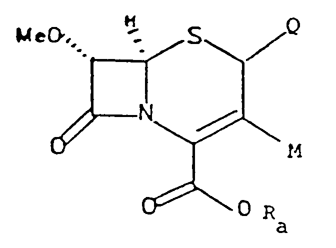 wherein Rₐ is a protecting group comprising C₁₋₆alkyl, benzyl and benzylhydryl;
(c) converting the ester formed in step (b) to a free acid of formula 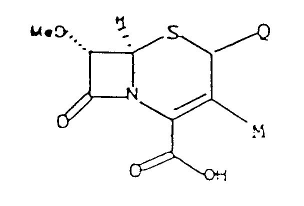
(d) reacting the free acid formed in step (c) with a heterocyclic compound of formula HB in the presence of N-hydroxysuccinimide, DCC and triethylamine to form the amide of formula 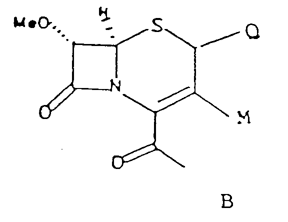 wherein B is defined previously; or alternatively esterifying the free acid of step (d) to form an ester of formula 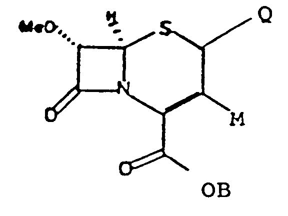 wherein B is as previously defined; and
(e) Oxidizing the product of step (d) with m-chloroperbenzoic acid or other suitable oxidation agents to form a sulfone of formula 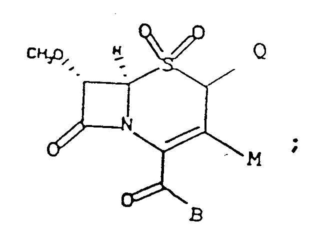 and optionally
(f) Converting the sulfone of step (e) to a compound of formula (I) by deblocking the protecting group.

9. The process of Claim 8 wherein the compound of formula 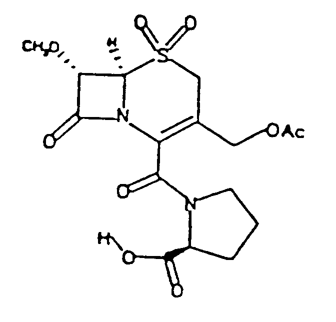 is prepared.

10. The intermediate formed by step (d) according to Claim 8 which is

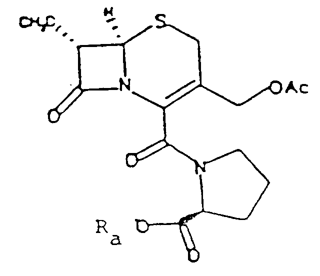

11. The intermediate formed by step (e) according to Claim 8 which is

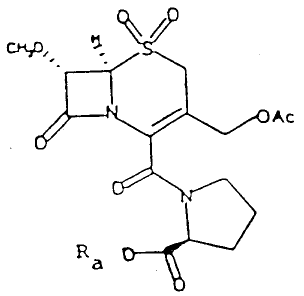

12. A pharmaceutical composition for treating elastase-mediated conditions in a mammalian species comprising a non-toxic pharmaceutical carrier and an effective amount of a compound of structural formula: 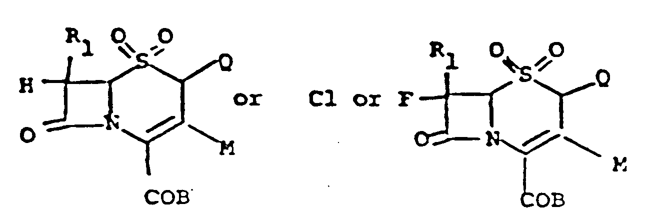 wherein M, R₁, B & Q are defined in claim 1.

13. The composition of Claim 12 wherein the active compound is 3-acetyloxymethyl-7α-methoxy-8-oxo-5-thia-1-azabicyclo[4·2·0·]oct-2-ene-2-(2-(S)-carboxypyrrolidinecarboxamide)-5,5-dioxide.

14. The use of a compound of structural formula: 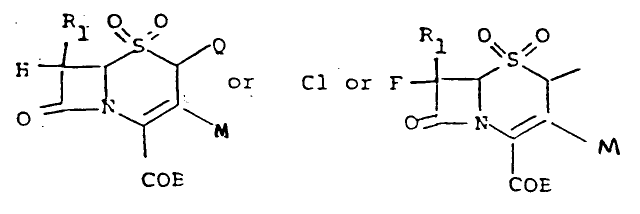 wherein M, R₁, B & Q are defined in claim 1.
for the preparation of a medicament useful for the treatment or management of elastase-mediated diseases.

## Claims (Claims for the following Contracting State(s): ES, GR)

1. A process for the preparation of a compound of structural formula: 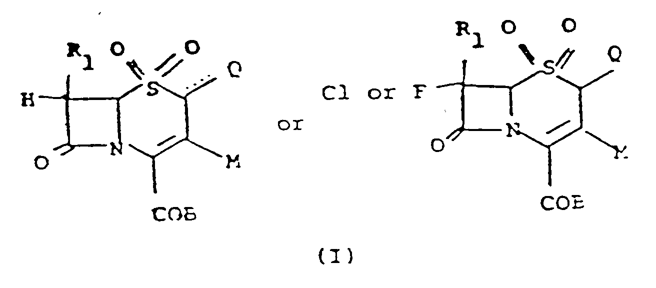 wherein M is:
(1) trifluoromethyl;
(2) chloro or fluoro;
(3) -COOH;
(4) -CHO; or
(5) -CH₂A wherein A represents
(a) hydrogen;
(b) halo;
(c) ZR₅ wherein Z is oxygen and R₅ is hydrogen or straight or branched C₁₋₃ alkyl,
(d) acyloxy wherein acyl is C₂₋₆ alkanoyl,
succinoyl, or
carbamoyl or thiocarbamoyl and N,-alkyl or N,N-dialkyl derivatives thereof in which alkyl groups contain 1-10 carbon atoms and may be further substituted by amino or carboxyl;
(f) acylthio wherein the acyl group is as defined in (d);
(g) 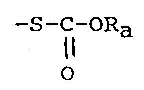 wherein Rₐ represents C₁₋₆ alkyl, phenyl or CH₂COOH;
(h) amino, acetamido, carbamoylamino, N,N-dimethyl amino, N-(2-chloroethyl)amino, 5-cyanotriazolyl, 4-methoxy carbonyl triazol-1-yl;
(i) Rₐ-SO- where Rₐ is C₁₋₁₆ alkyl or C₆₋₁₀ aryl
(j) Rₐ-SO₂- where Rₐ is defined in (i);
(k) a substituent selected from the group consisting of 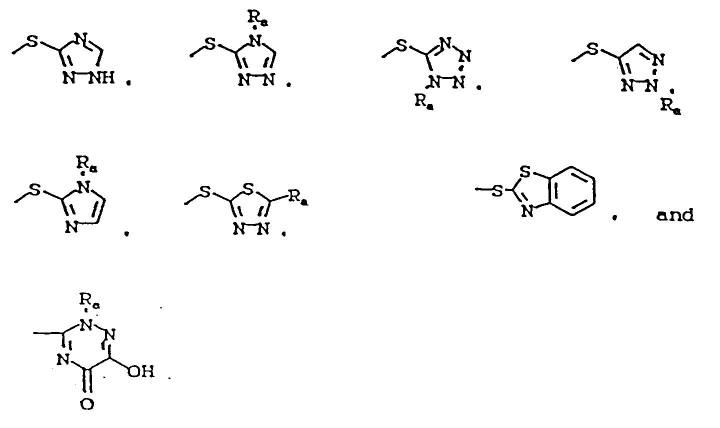 where Rₐ is C₁₋₁₆alkyl, C₆₋₁₀ aryl or CH₂CO₂H
(6) -CH=CH-Rₐ wherein Rₐ is as defined in (i);
R₁ is
(a) hydrogen;
(b) hydroxy;
(c) mercapto;
(d) OR¹₁ wherein R¹₁ is a hydrocarbyl group selected from a group consisting of straight or branched chain C₁₋₆ alkyl, C₃₋₆ alkenyl, C₃₋₆ alkynyl, phenyl and benzyl, these hydrocarbyl groups may be unsubstituted or substituted with one or more radicals which is hydroxy, halo, nitro, amino or carboxy;
(e) -SR¹₁ wherein R¹₁ is as previously defined;
(f) a hydrocarbyl group selected from a group consisting of
(1) straight or branched C₁₋₆ alkyl;
(2) straight or branched C₂₋₆ alkenyl;
(3) straight or branched C₂₋₆ alkynyl;
(4) benzyl or phenethyl;
(5) cyclopropyl or cyclopentyl;
(6) phenyl;
(7) -CF₃;
the above groups (1) to (6) can be unsubstituted or substituted with one or more radicals selected from a group consisting of halo, hydroxy, amino, nitro, sulfonyl, sylfamoyl, acyloxy, carbamoyloxy, carboxy or carboxamido;
(g) cyano;
(h) 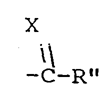 wherein X is oxygen or sulfur and R'' is
(1) hydrogen;
(2) hydroxy;
(3) mercapto;
(4) amine;
(5) N-alkyl or N,N-dialkylamine;
(6) C₁₋₆ alkyl;
(7) benzyloxy;
(8) phenoxy;
(9) C₁₋₆ alkylthio; or
(10) phenylthio;
(i) halo;
(j) 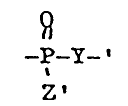 where Y' and Z' independently are OH, or -OC₁₋₆ alkyl;
(k) 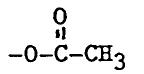
(l) -SO₂NH₂;
(m) -SO₂R₂ where R₂ is C₁₋₆ alkyl, halo C₁₋₆ alkyl, phenyl, or benzyl;
(n) -SO₂NR₃R₄ where R₃ and R₄ independently represent H or C₁₋₆ alkyl;
B is a heterocyclic group selected from the group comprising 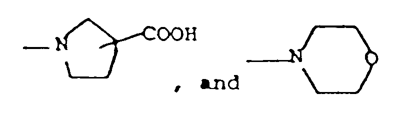 Q is
(1) hydrogen;
(2) C₁₋₆ alkyl;
(3) halo C₁₋₆ alkyl;
(4) hydroxy C₁₋₆ alkyl;
(5) methylene, C₁₋₆ alkylmethylene, phenyl methylene, phenylthiomethylene, phenylsulfinylmethylene or phenylsulfonyl methylene;
(6) C₁₋₆ alkoxy C₁₋₆ alkyl;
(7) benzyl;
(8) phenylthio C₁₋₆ alkyl, phenylsulfinyl C₁₋₆ alkyl or phenylsulfonyl C₁₋₆ alkyl;
(9) phenoxy C₁₋₆ alkyl; or
(10) phenylamino C₁₋₆ alkyl.

2. A process for preparing a compound as defined in Claim 1 comprising
(a) esterifying a compound of formula 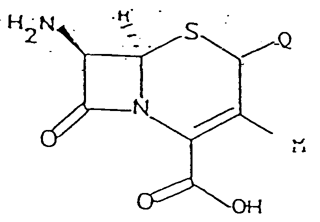 to form an ester of formula 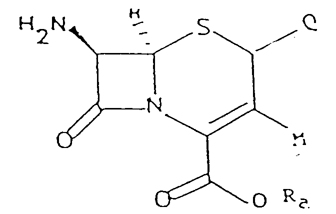 wherein Q and M are as previously defined; and Rₐ is a protecting group;
(b) diazotizing the ester of step (a) with NaNO₂ or other diazotizing reagents and converting the resulting diazo derivative with methanol and rhodium acetate dimer to form an ester of formula 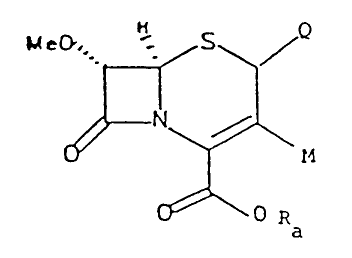 wherein Rₐ is a protecting group comprising C₁₋₆alkyl, benzyl and benzylhydryl;
(c) converting the ester formed in step (b) to a free acid of formula 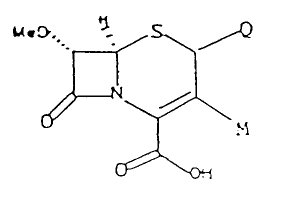
(d) reacting the free acid formed in step (c) with a heterocyclic compound of formula HB in the presence of N-hydroxysuccinimide, DCC and triethylamine to form the amide of formula 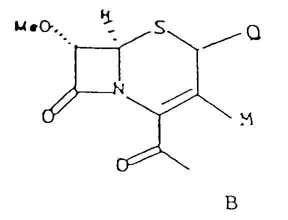 wherein B is defined previously; or alternatively esterifying the free acid of step (d) to form an ester of formula 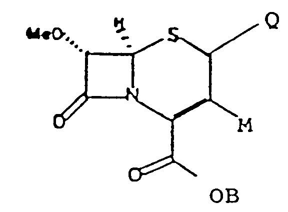 wherein B is as previously defined; and
(e) Oxidizing the product of step (d) with m-chloroperbenzoic acid or other suitable oxidation agents to form a sulfone of formula 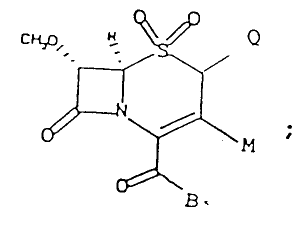 and optionally
(f) Converting the sulfone of step (e) to a compound of formula (I) by deblocking the protecting group.

3. The process of Claim 2 wherein the compound of formula 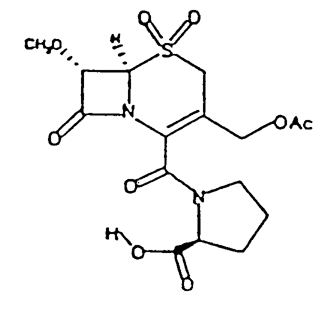 is prepared.

4. A process as claimed in claims 1 or 2 wherein a compound is prepared of the formula: 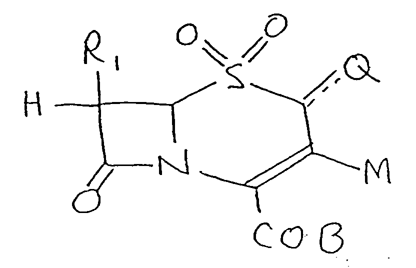 wherein M is:
(1) trifluoromethyl;
(2) chloro or fluoro; or
(3) -CH₂A wherein A represents:
(a) alkanoyloxy of 2-6 carbon atoms
(b) alkoxy of 1-3 carbon atoms
(c) halo
(d) hydrogen
(e) hydroxy
(f) mercapto or mercapto substituted by an aryl group of 2-6 carbon atoms
(g) carbamoyloxy
(h) -SO-CH₃ or - SO-C₆H₅
(i) -SO₂-CH₃ or -SO₂-C₆H₅
R₁ is:
(1) C₁₋₆ alkyl
(2) hydroxy
(3) OR: where R¹ is
(a) C₁₋₆ alkyl, C₃₋₆ alkenyl or C₃₋₆ alkynyl
(b) -C₆H₅
(c) CH₂CH₂C₆H₅ or
(d) -CO-R¹¹ where R¹¹ is hydrogen, C₁₋₆ alkyl, phenyl, benzyl or C₁₋₆ alkylamino
(4) fluoro or chloro
B is as defined in Claim 1 and
Q is
(1) hydrogen;
(2) methyl, ethyl or i- or n- propyl;
(3) methylene or
(4) phenylthiomethyl or phenyl sulfonylmethyl.

5. A process as claimed in claim 4 wherein Q is hydrogen.

6. A process as claimed in claims 4 or 5 wherein R₁ is methoxy.

7. A process as claimed in any of claims 4 to 6 wherein M is CH₂A wherein A is alkanoyloxy.

## Patentansprüche (Patentansprüche für folgende(n) Vertragsstaat(en): DE, GB, FR, IT, NL, SE, CH, LI, BE, AT, LU)

1. Eine Verbindung der Strukturformel: 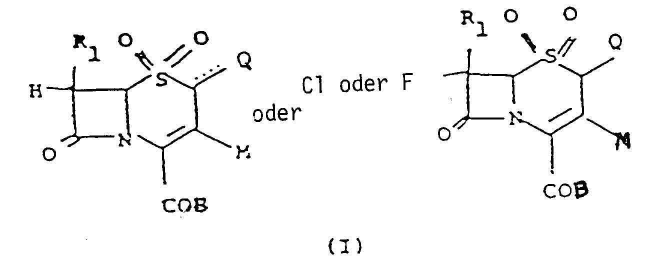 wobei M:
(1) Trifluormethyl;
(2) Chlor oder Fluor;
(3) -COOH;
(4) -CHO oder
(5) -CH₂A, wobei A
(a) Wasserstoff;
(b) Halogen;
(c) ZR₅, wobei Z Sauerstoff und R₅ Wasserstoff oder gerades oder verzweigtes C₁₋₃-Alkyl ist,
(d) Acyloxy, wobei Acyl C₂₋₆-Alkanoyl, Succinoyl oder Carbamoyl oder Thiocarbamoyl und deren N,-Alkyl oder N,N-Dialkyl-Derivate ist, bei denen die Alkylgruppen 1-10 Kohlenstoffatome enthalten und durch Amino oder Carboxyl weiter substituiert sein können;
(f) Acylthio, wobei die Acylgruppe wie in (d) definiert ist;
(g) 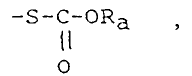 wobei Rₐ C₁₋₆-Alkyl, Phenyl oder CH₂COOH darstellt;
(h) Amino, Acetamido, Carbamoylamino, N,N-Dimethylamino, N-(2-Chlorethyl)amino, 5-Cyanotriazolyl, 4-Methoxycarbonyltriazol-1-yl;
(i) Rₐ-SO-, wobei Rₐ C₁₋₁₆-Alkyl oder C₆₋₁₀-Aryl ist;
(j) Rₐ-SO₂-, wobei Rₐ wie in (i) definiert ist;
(k) einen Substituenten darstellt, der ausgewählt ist aus der Gruppe, bestehend aus 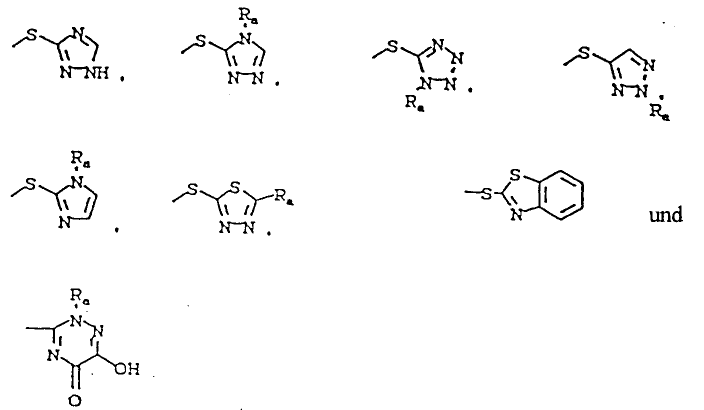 wobei Rₐ C₁₋₁₆-Alkyl, C₆₋₁₀-Aryl oder CH₂CO₂H ist;
(6) -CH=CH-Rₐ ist, wobei Rₐ wie in (i) definiert ist;
R₁
(a) Wasserstoff;
(b) Hydroxy;
(c) Mercapto;
(d) OR¹₁, wobei R¹₁ eine Kohlenwasserstoffgruppe ist, die ausgewählt ist aus einer Gruppe, die aus geradkettigem oder verzweigtkettigem C₁₋₆-Alkyl, C₃₋₆-Alkenyl, C₃₋₆-Alkinyl, Phenyl und Benzyl besteht, wobei diese Kohlenwasserstoffgruppen unsubstituiert oder mit einem oder mehreren Resten substituiert sein können, welche Hydroxy, Halogen, Nitro, Amino oder Carboxy sind;
(e) -SR¹₁, wobei R¹₁ wie vorstehend definiert ist;
(f) eine Kohlenwasserstoffgruppe, die ausgewählt ist aus einer Gruppe, bestehend aus
(1) geradem oder verzweigtem C₁₋₆-Alkyl;
(2) geradem oder verzweigtem C₂₋₆-Alkenyl;
(3) geradem oder verzweigtem C₂₋₆-Alkinyl;
(4) Benzyl oder Phenethyl;
(5) Cyclopropyl oder Cyclopentyl;
(6) Phenyl;
(7) -CF₃,
wobei die vorstehenden Gruppen (1) bis (6) unsubstituiert oder mit einem oder mehreren Resten substituiert sein können, die ausgewählt sind aus einer Gruppe, die aus Halogen, Hydroxy, Amino, Nitro, Sulfonyl, Sulfamoyl, Acyloxy, Carbamoyloxy, Carboxy oder Carboxamido besteht;
(g) Cyano;
(h) 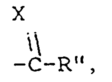 wobei X Sauerstoff oder Schwefel ist und R''
(1) Wasserstoff;
(2) Hydroxy;
(3) Mercapto;
(4) Amin;
(5) N-Alkyl- oder N,N-Dialkylamin;
(6) C₁₋₆-Alkyl;
(7) Benzyloxy;
(8) Phenoxy;
(9) C₁₋₆-Alkylthio oder
(10) Phenylthio ist;
(i) Halogen;
(j) 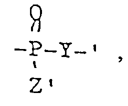 wobei Y' und Z' unabhängig voneinander OH oder -OC₁₋₆-Alkyl sind;
(k) 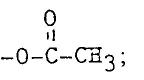
(l) -SO₂NH₂;
(m) -SO₂R₂, wobei R₂ C₁₋₆-Alkyl, Halogen-C₁₋₆-alkyl, Phenyl oder Benzyl ist;
(n) -SO₂NR₃R₄ ist, wobei R₃ und R₄ unabhängig voneinander H oder C₁₋₆-Alkyl darstellen;
B eine heterocyclische Gruppe ist, die ausgewählt ist aus der Gruppe, umfassend: 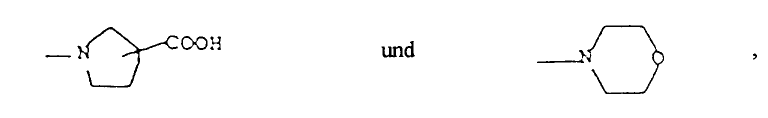 Q
(1) Wasserstoff;
(2) C₁₋₆-Alkyl;
(3) Halogen-C₁₋₆-alkyl;
(4) Hydroxy-C₁₋₆-alkyl;
(5) Methylen, C₁₋₆-Alkylmethylen, Phenylmethylen, Phenylthiomethylen, Phenylsulfinylmethylen oder Phenylsulfonylmethylen;
(6) C₁₋₆-Alkoxy-C₁₋₆-alkyl;
(7) Benzyl;
(8) Phenylthio-C₁₋₆-alkyl, Phenylsulfinyl-C₁₋₆-alkyl oder Phenylsulfonyl-C₁₋₆-alkyl;
(9) Phenoxy-C₁₋₆-alkyl oder
(10) Phenylamino-C₁₋₆-alkyl.

2. Die Verbindung nach Anspruch 1, die 3-Acetyloxymethyl-7α-methoxy-8-oxo-5-thia-1-azabicyclo[4.2.0]oct-2-en-2-(2-(S)-carboxypyrrolidincarboxamid)-5,5-dioxid ist.

3. Eine Verbindung der Formel: 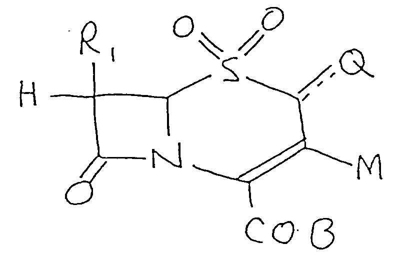 wobei M
(1) Trifluormethyl;
(2) Chlor oder Fluor oder
(3) -CH₂A ist, wobei A
(a) Alkanoyloxy mit 2-6 Kohlenstoffatomen;
(b) Alkoxy mit 1-3 Kohlenstoffatomen;
(c) Halogen;
(d) Wasserstoff;
(e) Hydroxy;
(f) Mercapto oder mit einer Arylgruppe mit 2-6 Kohlenstoffatomen substituiertes Mercapto;
(g) Carbamoyloxy;
(h) -SO-CH₃ oder -SO-C₆H₅;
(i) -SO₂-CH₃ oder -SO₂-C₆H₅
darstellt,
R₁
(1) C₁₋₆-Alkyl;
(2) Hydroxy;
(3) OR, wobei R₁
(a) C₁₋₆-Alkyl, C₃₋₆-Alkenyl, C₃₋₆-Alkinyl,
(b) -C₆H₅,
(c) CH₂CH₂C₆H₅ oder
(d) -CO-R¹¹ ist, wobei R¹¹ Wasserstoff, C₁₋₆-Alkyl, Phenyl, Benzyl oder C₁₋₆-Alkylamino ist,
(4) Fluor oder Chlor ist,
B wie in Anspruch 1 definiert ist, und
Q
(1) Wasserstoff;
(2) Methyl, Ethyl oder i- oder n-Propyl;
(3) Methylen oder
(4) Phenylthiomethyl oder Phenylsulfonylmethyl ist.

4. Eine Verbindung, wie in Anspruch 3 beansprucht, wobei Q Wasserstoff ist.

5. Eine Verbindung, wie in Anspruch 3 oder 4 beansprucht, wobei R₁ Methoxy ist.

6. Eine Verbindung, wie in einem der Ansprüche 3 bis 5 beansprucht, wobei M CH₂A ist, wobei A Alkanoyloxy ist.

7. Ein Verfahren zur Herstellung einer Verbindung nach Anspruch 1 mit der Strukturformel (I) 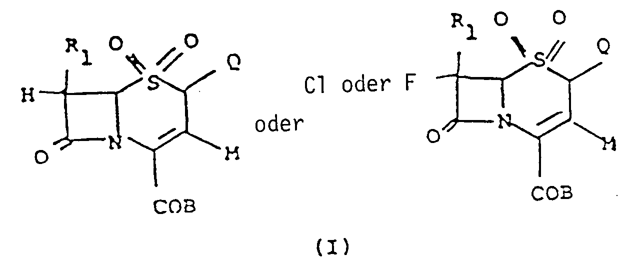 wobei M, R₁, B & Q wie in Anspruch 1 definiert sind, umfassend
(a) Behandeln einer Verbindung der Formel 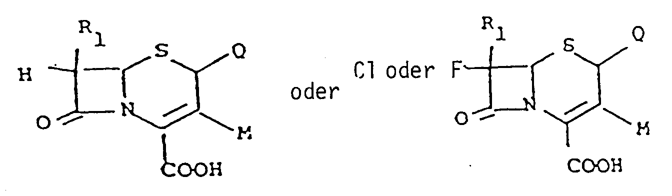 mit einer Verbindung der Formel HB in der Gegenwart eines Katalysators; und
(b) Oxidieren des Produktes aus Schritt (a) zu einer Verbindung der Formel (I).

8. Ein Verfahren zur Herstellung einer Verbindung nach Anspruch 1, umfassend
(a) Verestern einer Verbindung der Formel 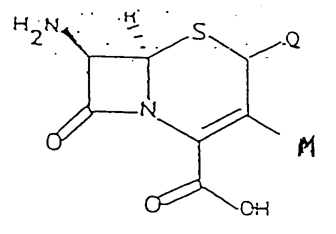 unter Bildung eines Esters der Formel 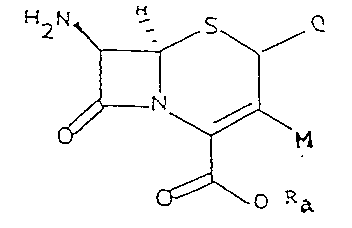 wobei Q und M wie vorher definiert sind und Rₐ eine Schutzgruppe ist;
(b) Diazotieren des Esters aus Schritt (a) mit NaNO₂ oder anderen Diazotierungsreagenzien und Umwandeln des entstehenden Diazoderivates mit Methanol und Rhodiumacetat-Dimer unter Bildung eines Esters der Formel 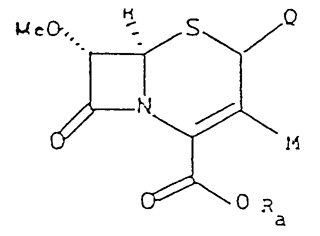 wobei Rₐ eine Schutzgruppe ist, die C₁₋₆-Alkyl, Benzyl und Benzylhydryl umfaßt;
(c) Umwandeln des in Schritt (b) gebildeten Esters in eine freie Säure der Formel 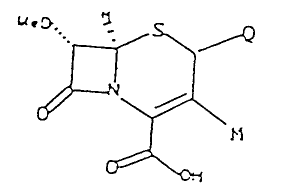
(d) Umsetzen der in Schritt (c) gebildeten freien Säure mit einer heterocyclischen Verbindung der Formel HB in der Gegenwart von N-Hydroxysuccinimid, DCC und Triethylamin unter Bildung des Amids der Formel 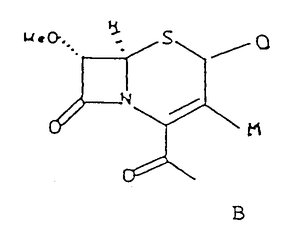 wobei B vorstehend definiert ist; oder alternativ Verestern der freien Säure aus Schritt (d) unter Bildung eines Esters der Formel 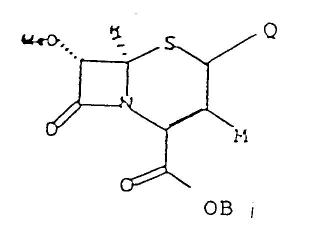 wobei B wie vorher definiert ist, und
(e) Oxidieren des Produktes aus Schritt (d) mit m-Chlorperbenzoesäure oder anderen geeigneten Oxidationsmitteln unter Bildung eines Sulfons der Formel 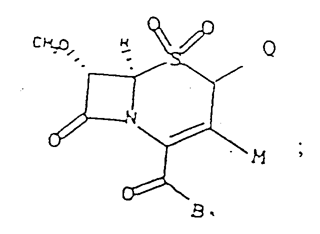 und wahlweise
(f) Umwandeln des Sulfons aus Schritt (e) in eine Verbindung der Formel (I) durch Entfernen der Schutzgruppe.

9. Das Verfahren nach Anspruch 8, wobei die Verbindung der Formel 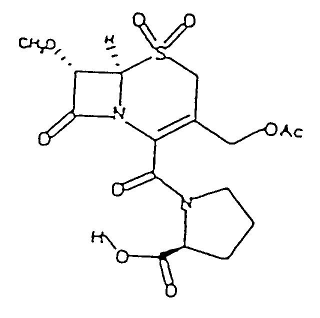 hergestellt wird.

10. Das Zwischenprodukt, das durch Schritt (d) nach Anspruch 8 gebildet wird, welches 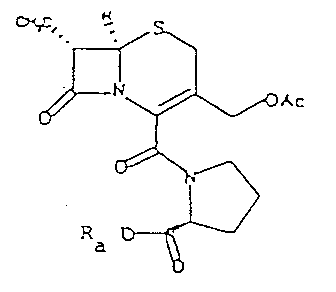 ist.

11. Das Zwischenprodukt, das durch Schritt (e) nach Anspruch 8 gebildet wird, welches 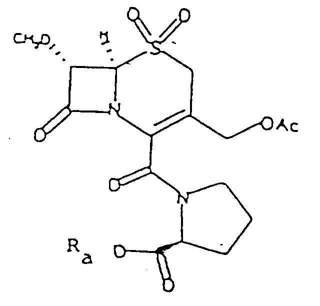 ist.

12. Eine pharmazeutische Zusammensetzung zur Behandlung Elastase-vermittelter Zustände bei einer Säugerspezies, umfassend einen nicht toxischen pharmazeutischen Träger und eine wirksame Menge einer Verbindung der Strukturformel: 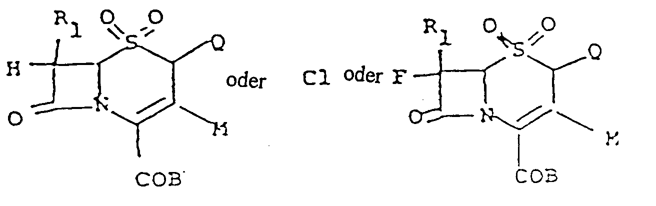 wobei M, R₁, B & Q wie in Anspruch 1 definiert sind.

13. Die Zusammensetzung nach Anspruch 12, wobei die aktive Verbindung 3-Acetyloxymethyl-7α-methoxy-8-oxo-5-thia-1-azabicyclo[4.2.0]oct-2-en-2-(2-(S)-carboxypyrrolidincarboxamid)-5,5-dioxid ist.

14. Die Verwendung einer Verbindung der Strukturformel: 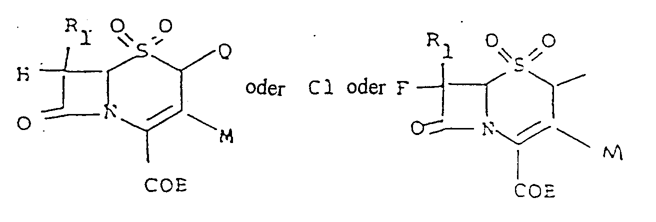 wobei M, R₁, B & Q wie in Anspruch 1 definiert sind, für die Herstellung eines Arzneimittels, das für die Behandlung oder Handhabung von Elastase-vermittelten Krankheiten nützlich ist.

## Patentansprüche (Patentansprüche für folgende(n) Vertragsstaat(en): ES, GR)

1. Ein Verfahren für die Herstellung einer Verbindung der Strukturformel: 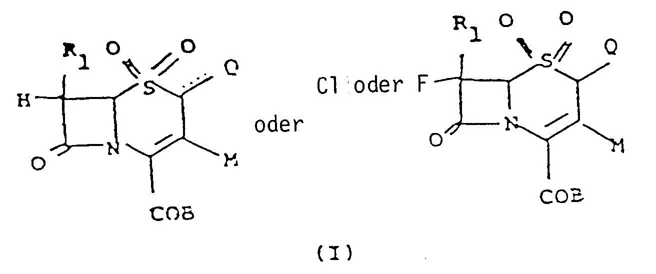 wobei M:
(1) Trifluormethyl;
(2) Chlor oder Fluor;
(3) -COOH;
(4) -CHO oder
(5) -CH₂A, wobei A
(a) Wasserstoff;
(b) Halogen;
(c) ZR₅, wobei Z Sauerstoff und R₅ Wasserstoff oder gerades oder verzweigtes C₁₋₃-Alkyl ist,
(d) Acyloxy, wobei Acyl C₂₋₆-Alkanoyl, Succinoyl oder Carbamoyl oder Thiocarbamoyl und deren N,-Alkyl oder N,N-Dialkyl-Derivate ist, bei denen die Alkylgruppen 1-10 Kohlenstoffatome enthalten und durch Amino oder Carboxyl weiter substituiert sein können;
(f) Acylthio, wobei die Acylgruppe wie in (d) definiert ist;
(g) 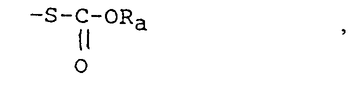 wobei Rₐ C₁₋₆-Alkyl, Phenyl oder CH₂COOH darstellt;
(h) Amino, Acetamido, Carbamoylamino, N,N-Dimethylamino, N-(2-Chlorethyl)amino, 5-Cyanotriazolyl, 4-Methoxycarbonyltriazol-1-yl;
(i) Rₐ-SO-, wobei Rₐ C₁₋₁₆-Alkyl oder C₆₋₁₀-Aryl ist;
(j) Rₐ-SO₂-, wobei Rₐ wie in (i) definiert ist;
(k) einen Substituenten darstellt, der ausgewählt ist aus der Gruppe, bestehend aus 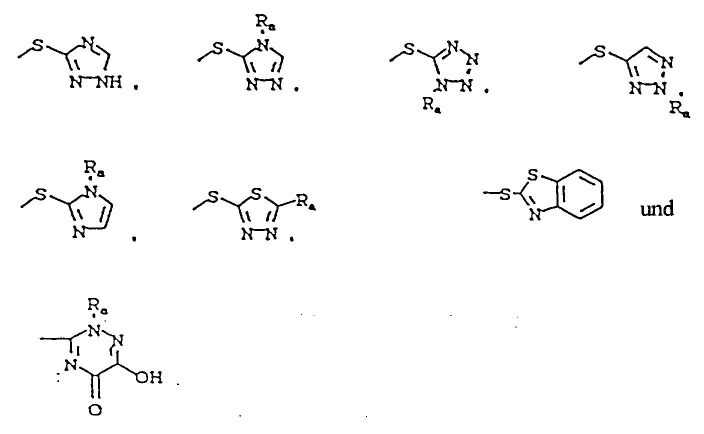 wobei Rₐ C₁₋₁₆-Alkyl, C₆₋₁₀-Aryl oder CH₂CO₂H ist;
(6) -CH=CH-Rₐ ist, wobei Rₐ wie in (i) definiert ist;
R₁
(a) Wasserstoff;
(b) Hydroxy;
(c) Mercapto;
(d) OR¹₁, wobei R¹₁ eine Kohlenwasserstoffgruppe ist, die ausgewählt ist aus einer Gruppe, die aus geradkettigem oder verzweigtkettigem C₁₋₆-Alkyl, C₃₋₆-Alkenyl, C₃₋₆-Alkinyl, Phenyl und Benzyl besteht, wobei diese Kohlenwasserstoffgruppen unsubstituiert oder mit einem oder mehreren Resten substituiert sein können, welche Hydroxy, Halogen, Nitro, Amino oder Carboxy sind;
(e) -SR¹₁, wobei R¹₁ wie vorstehend definiert ist;
(f) eine Kohlenwasserstoffgruppe, die ausgewählt ist aus einer Gruppe, bestehend aus
(1) geradem oder verzweigtem C₁₋₆-Alkyl;
(2) geradem oder verzweigtem C₂₋₆-Alkenyl;
(3) geradem oder verzweigtem C₂₋₆-Alkinyl;
(4) Benzyl oder Phenethyl;
(5) Cyclopropyl oder Cyclopentyl;
(6) Phenyl;
(7) -CF_{3,}
wobei die vorstehenden Gruppen (1) bis (6) unsubstituiert oder mit einem oder mehreren Resten substituiert sein können, die ausgewählt sind aus einer Gruppe, die aus Halogen, Hydroxy, Amino, Nitro, Sulfonyl, Sulfamoyl, Acyloxy, Carbamoyloxy, Carboxy oder Carboxamido besteht;
(g) Cyano;
(h) 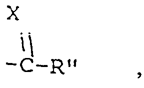 wobei X Sauerstoff oder Schwefel ist und R''
(1) Wasserstoff;
(2) Hydroxy;
(3) Mercapto;
(4) Amin;
(5) N-Alkyl- oder N,N-Dialkylamin;
(6) C₁₋₆-Alkyl;
(7) Benzyloxy;
(8) Phenoxy;
(9) C₁₋₆-Alkylthio oder
(10) Phenylthio ist;
(i) Halogen;
(j) 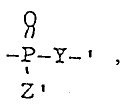 wobei Y' und Z' unabhängig voneinander OH oder -OC₁₋₆-Alkyl sind;
(k) 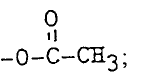
(l) -SO₂NH₂;
(m) -SO₂R₂, wobei R₂ C₁₋₆-Alkyl, Halogen-C₁₋₆-alkyl, Phenyl oder Benzyl ist;
(n) -SO₂NR₃R₄ ist, wobei R₃ und R₄ unabhängig voneinander H oder C₁₋₆-Alkyl darstellen;
B eine heterocyclische Gruppe ist, die ausgewählt ist aus der Gruppe, umfassend: 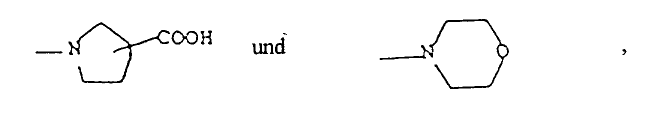 Q
(1) Wasserstoff;
(2) C₁₋₆-Alkyl;
(3) Halogen-C₁₋₆-alkyl;
(4) Hydroxy-C₁₋₆-alkyl;
(5) Methylen, C₁₋₆-Alkylmethylen, Phenylmethylen, Phenylthiomethylen, Phenylsulfinylmethylen oder Phenylsulfonylmethylen;
(6) C₁₋₆-Alkoxy-C₁₋₆-alkyl;
(7) Benzyl;
(8) Phenylthio-C₁₋₆-alkyl, Phenylsulfinyl-C₁₋₆-alkyl oder Phenylsulfonyl-C₁₋₆-alkyl;
(9) Phenoxy-C₁₋₆-alkyl oder
(10) Phenylamino-C₁₋₆-alkyl
ist.

2. Ein Verfahren zur Herstellung einer Verbindung, wie in Anspruch 1 definiert, umfassend
(a) Verestern einer Verbindung der Formel 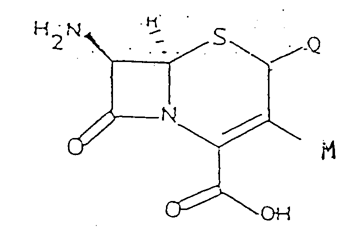 unter Bildung eines Esters der Formel 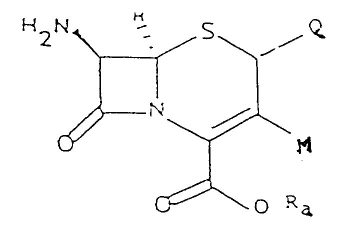 wobei Q und M wie vorher definiert sind und Rₐ eine Schutzgruppe ist;
(b) Diazotieren des Esters aus Schritt (a) mit NaNO₂ oder anderen Diazotierungsreagenzien und Umwandeln des entstehenden Diazoderivates mit Methanol und Rhodiumacetat-Dimer unter Bildung eines Esters der Formel 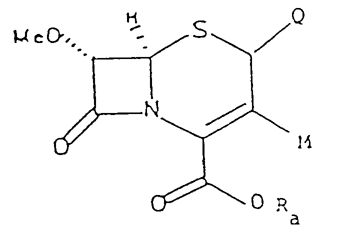 wobei Rₐ eine Schutzgruppe ist, die C₁₋₆-Alkyl, Benzyl und Benzylhydryl umfaßt;
(c) Umwandeln des in Schritt (b) gebildeten Esters in eine freie Säure der Formel 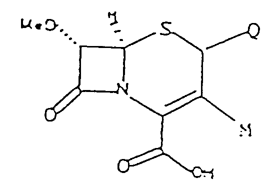
(d) Umsetzen der in Schritt (c) gebildeten freien Säure mit einer heterocyclischen Verbindung der Formel HB in der Gegenwart von N-Hydroxysuccinimid, DCC und Triethylamin unter Bildung des Amids der Formel 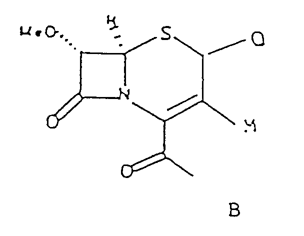 wobei B vorstehend definiert ist; oder alternativ Verestern der freien Säure aus Schritt (d) unter Bildung eines Esters der Formel 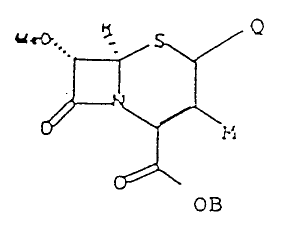 wobei B wie vorher definiert ist; und
(e) Oxidieren des Produktes aus Schritt (d) mit m-Chlorperbenzoesäure oder anderen geeigneten Oxidationsmitteln unter Bildung eines Sulfons der Formel 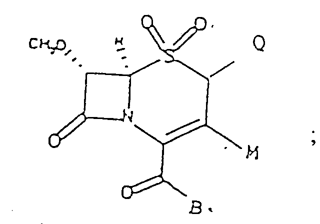 und wahlweise
(f) Umwandeln des Sulfons aus Schritt (e) in eine Verbindung der Formel (I) durch Entfernen der Schutzgruppe.

3. Das Verfahren nach Anspruch 2, wobei die Verbindung der Formel 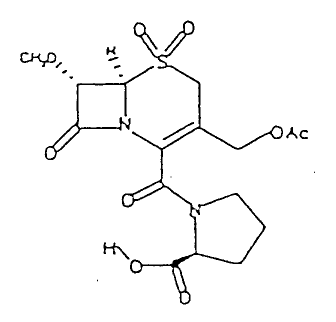 hergestellt wird.

4. Ein Verfahren, wie beansprucht in Anspruch 1 oder 2, wobei eine Verbindung der Formel: 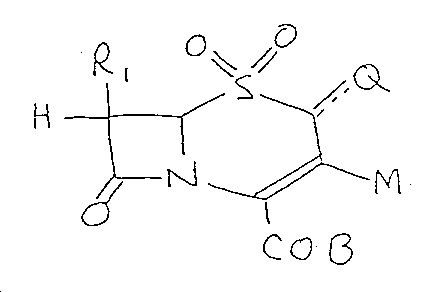 hergestellt wird, wobei
M
(1) Trifluormethyl;
(2) Chlor oder Fluor; oder
(3) -CH₂A ist, wobei A
(a) Alkanoyloxy mit 2-6 Kohlenstoffatomen;
(b) Alkoxy mit 1-3 Kohlenstoffatomen;
(c) Halogen;
(d) Wasserstoff;
(e) Hydroxy;
(f) Mercapto oder mit einer Arylgruppe mit 2-6 Kohlenstoffatomen substituiertes Mercapto;
(g) Carbamoyloxy;
(h) -SO-CH₃ oder -SO-C₆H₅;
(i) -SO₂-CH₃ oder -SO₂-C₆H₅ darstellt,
R₁
(1) C₁₋₆-Alkyl;
(2) Hydroxy;
(3) OR, wobei R¹
(a) C₁₋₆-Alkyl, C₃₋₆-Alkenyl oder C₃₋₆-Alkinyl,
(b) -C₆H₅,
(c) CH₂CH₂C₆H₅ oder
(d) -CO-R¹¹ ist, wobei R¹¹ Wasserstoff, C₁₋₆-Alkyl, Phenyl, Benzyl oder C₁₋₆-Alkylamino ist,
(4) Fluor oder Chlor ist,
B wie in Anspruch 1 definiert ist, und
Q
(1) Wasserstoff;
(2) Methyl, Ethyl oder i- oder n-Propyl;
(3) Methylen oder
(4) Phenylthiomethyl oder Phenylsulfonylmethyl ist.

5. Ein Verfahren, wie in Anspruch 4 beansprucht, wobei Q Wasserstoff ist.

6. Ein Verfahren, wie in Anspruch 4 oder 5 beansprucht, wobei R₁ Methoxy ist.

7. Ein Verfahren, wie in einem der Ansprüche 4 bis 6 beansprucht, wobei M CH₂A ist, wobei A Alkanoyloxy ist.

## Revendications (Revendications pour l'(les) Etat(s) contractant(s) suivant(s): DE, GB, FR, IT, NL, SE, CH, LI, BE, AT, LU)

1. Un composé de la formule structurelle 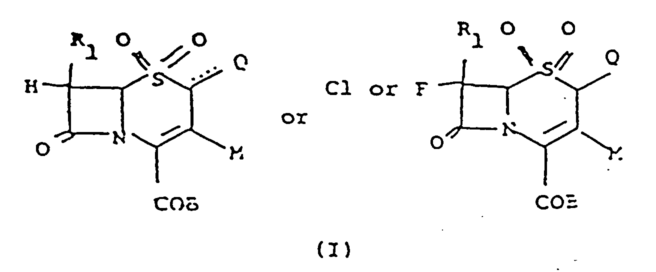 où M est:
(1) trifluorométhyle;
(2) chlore ou fluor;
(3) -COOH;
(4) -CHO; ou
(5) -CH₂A où A représente
(a) de l'hydrogène;
(b) de l'halogène;
(c) ZR₅ où Z est de l'oxygène et R₅ est de l'hydrogène ou un alkyle en C₁ à C₃, à chaîne droite ou ramifiée,
d) un acyloxy où l'acyle est un alcanoyle en C₂ à C₆, un succinoyle, ou bien un carbamoyle ou thiocarbamoyle et des dérivés N-alkyle ou N,N-dialkyle de ceux-ci dans lesquels les groupes alkyle renferment de 1 à 10 atomes de carbone et peuvent être en outre substitués par un amino ou un carboxyle;
(f) un acylthio dans lequel le groupe acyle est tel que défini au point (d);
(g) 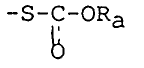 où Rₐ représente un acyle en C₁ à C₆, un phényle ou CH₂COOH;
(h) un radical amino, acétamido, carbamoylamino, N,N-diméthyl-amino, N-(2-chloroéthyl)amino, 5-cyanotriazolyle, 4-méthoxy carbonyl-triazol-1-yle;
(i) Rₐ-SO- où Rₐ est un alkyle en C₁ à C₁₆ ou un aryle en C₆ à C₁₀,
(j) Rₐ-SO₂- où Rₐ est défini au point (i);
(k) un substituant choisi dans le groupe constitué de 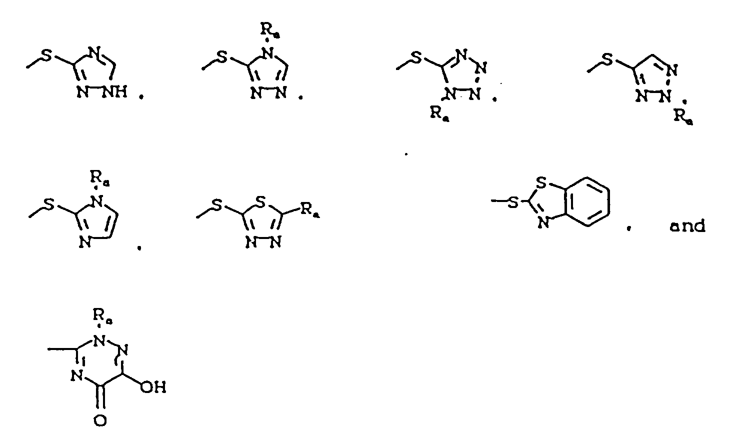 où Rₐ est un alkyle en C₁ à C₁₆, un aryle en C₆ à C₁₀ ou CH₂CO₂H;
(6) -CH=CH-Rₐ où Rₐ est tel que défini au point (i);
R₁ est
(a) de l'hydrogène;
(b) un radical hydroxy;
(c) un mercapto;
(d) OR¹₁ où R¹₁ est un groupe hydrocarbyle choisi dans un groupe constitué par un alkyle en C₁ à C₆, à chaîne droite ou ramifiée, un alcényle en C₃ à C₆, un alcynyle en C₃ à C₆, du phényle et du benzyle, ces groupes hydrocarbyle peuvent être non substitués ou substitués par un ou plus d'un radical qui est un hydroxy, de l'halogène, un nitro, un radical amino ou carboxy;
(e) -SR¹₁ où R¹₁ est tel que défini auparavant;
(f) un groupe hydrocarbyle choisi dans un groupe constitué de
(1) un alkyle en C₁ à C₆, à chaîne droite ou ramifiée;
(2) un alcényle en C₂ à C₆ à chaîne droite ou ramifiée;
(3) un alcynyle en C₂ à C₆ à chaîne droite ou ramifiée;
(4) du benzyle ou du phénéthyle;
(5) du cyclopropyle ou du cyclopentyle;
(6) du phényle;
(7) -CF₃;
les groupes ci-dessus (1) à (6) peuvent être non substitués ou bien substitués par un ou plus d'un radical choisi dans un groupe constitué par les radicaux halo, hydroxy, amino, nitro, sulfonyle, sulfamoyle, acyloxy, carbamoyloxy, carboxy ou carboxamido;
(g) du cyano;
(h) 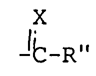 où X est de l'oxygène ou du soufre et R'' est
(1) de l'hydrogène;
(2) de l'hydroxy;
(3) un radical mercapto;
(4) un radical amine;
(5) un radical N-alcyle ou N,N-dialkylamine;
(6) un alkyle en C₁ à C₆;
(7) du benzyloxy;
(8) du phénoxy;
(9) un alkylthio en C₁ à C₆; ou
(10) un radical phénylthio;
(i) un radical halo;
(j) 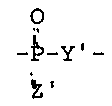 où Y' et Z' sont indépendamment OH, ou un radical -O-alkyle en C₁ à C₆;
(k) 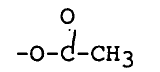
(l) -SO₂NH₂;
(m) -SO₂R₂ où R₂ est un radical alkyle en C₁ à C₆, halo-alkyle en C₁ à C₆, phényle ou benzyle;
(n) -SO₂NR₃R₄ où R₃ et R₄ représentent de façon indépendante, H ou un alkyle en C₁ à C₆;
B est un groupe hétérocyclique choisi dans le groupe comportant 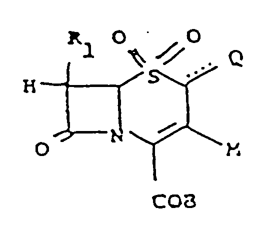 Q est
(1) de l'hydrogène;
(2) un alkyle en C₁ à C₆;
(3) un radical halo-alkyle en C₁ à C₆;
(4) un radical hydroxy-alkyle en C₁ à C₆;
(5) un radical méthylène, alkyl (en C₁ à C₆)méthylène, phényl-méthylène, phénylthio-méthylène, phénylsulfinyl-méthylène, ou phénylsulfonyl-méthylène;
(6) un alcoxy (en C₁ à C₆)-alkyl(en C₁ à C₆);
(7) du benzyle; (8) un radical phénylthio-alkyle en C₁ à C₆,
phénylsulfinyl-alkyle en C₁ à C₆ ou phénylsulfonyl-alkyle en C₁ à C₆;
(9) un radical phénoxy-alkyle en C₁ à C₆; ou
(10) un radical phénylamino-alkyle en C₁ à C₆.

2. Le composé de la revendication 1 qui est le 3-acétyloxyméthyl-7α-méthoxy-8-oxo-5-thia-1-azabicyclo[ 4.2.0]oct-2-ène-2-(2-(S)-carboxypyrrolidinecarboxamide)-5,5-dioxyde.

3. Un composé de la formule 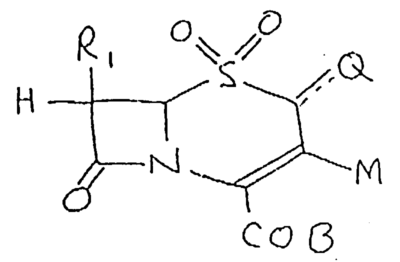 dans laquelle M est:
(1) du trifluorométhyle;
(2) un radical chloro ou fluoro; ou
(3) -CH₂A où A représente:
(a) un alcanoyloxy de 2 à 6 atomes de carbone;
(b) un alcoxy de 1 à 3 atomes de carbone;
(c) un radical halo;
(d) de l'hydrogène;
(e) de l'hydroxy;
(f) un radical mercapto ou mercapto substitué par un groupe aryle de 2 à 6 atomes de carbone;
(g) du carbamoyloxy;
(h) -SO-CH₃ ou -SO-C₆H₅
(i) -SO₂-CH₃ ou -SO₂-C₆H₅;
R₁ est :
(1) un alkyle en C₁ à C₆;
(2) de l'hydroxy;
(3) OR: où R¹ est
(a) un radical alkyle en C₁ à C₆, alcényle en C₃ à C₆ ou alcynyle en C₃ à C₆;
(b) -C₆H₅;
(c) CH₂CH₂C₆H₅ ou
(d) -CO-R¹¹ où R¹¹ est de l'hydrogène, un radical alkyle en C₁ à C₆, phényle, benzyle ou alkylamino en C₁ à C₆;
(4) un radical fluoro ou chloro;
B est tel que défini dans la revendication 1 et Q est
(1) de l'hydrogène;
(2) un radical méthyle, éthyle ou bien i- ou n-propyle;
(3) du méthylène ou
(4) un radical phénylthiométhyle ou phénylsulfonylméthyle.

4. Un composé tel que revendiqué dans la revendication 3, dans lequel Q est de l'hydrogène.

5. Un composé tel que revendiqué dans la revendication 3 ou 4, dans lequel R₁ est du méthoxy.

6. Un composé tel que revendiqué dans l'une quelconque des revendications 3 à 5, dans lequel M est CH₂A, où A est un alcanoyloxy.

7. Un procédé pour la préparation d'un composé selon la revendication 1 ayant les formules structurelles (I) 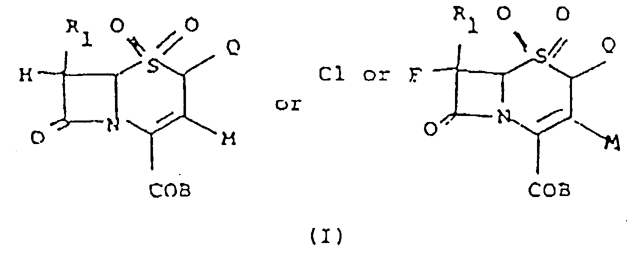 dans lesquelles M, R₁, B et Q sont définis comme dans la revendication 1, comportant (a) le traitement d'un composé de formule 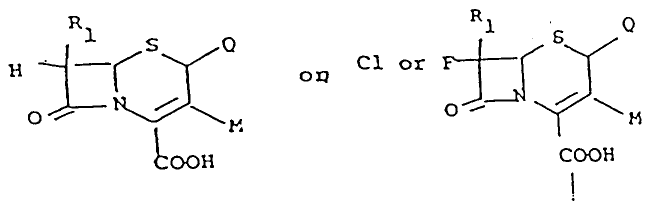 avec un composé de formule HB en présence d'un catalyseur et (b) l'oxydation du produit provenant de l'étape (a) en un composé de formule (I).

8. Un procédé pour préparer un composé selon la revendication 1, comportant
(a) l'estérification d'un composé de formule 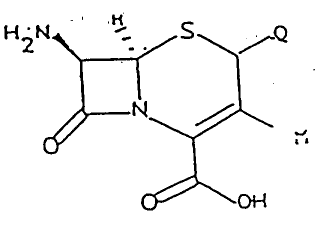 afin d'obtenir un ester de formule: 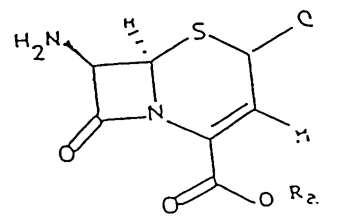 dans laquelle Q et M sont tels que définis auparavant; et Rₐ est un groupe protecteur;
(b) la diazotation de l'ester de l'étape (a) avec du NaNO₂ ou d'autres réactifs de diazotation et la conversion du dérivé diazoté obtenu avec du méthanol et de l'acétate de rhodium dimère pour former un ester de la formule 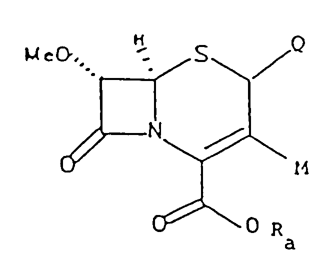 dans laquelle Rₐ est un groupe protecteur comportant un radical alkyle en C₁ à C₆, benzyle et benzylhydryle;
(c) la conversion de l'ester formé à l'étape (b) en un acide libre de formule: 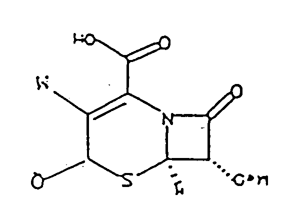
(d) la réaction de l'acide libre formé à l'étape (c) avec un composé hétérocyclique de formule HB en présence de N-hydroxysuccinimide, de DCC et de triéthylamine afin de former l'amide de formule 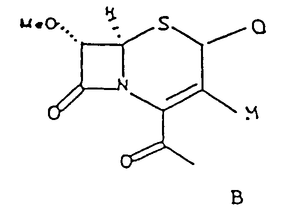 dans laquelle B est défini auparavant; ou, en variante l'estérification de l'acide libre de l'étape (d) pour former un ester de la formule: 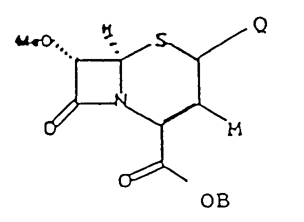 dans laquelle B est tel que défini auparavant; et
(e) l'oxydation du produit de l'étape (d) avec de l'acide m-chloroperbenzoïque ou d'autres agents d'oxydation appropriés afin de former une sulfone de formule 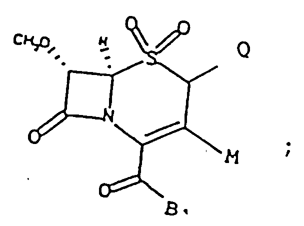 et, le cas échéant
(f) la conversion de la sulfone de l'étape (e) en un composé de formule (I) par déblocage du groupe protecteur.

9. Le procédé de la revendication 8, dans lequel le composé de formule 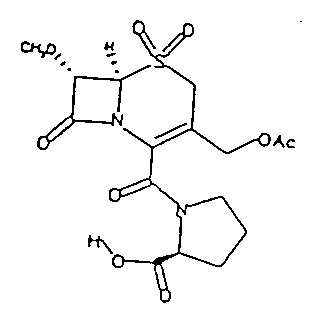 est préparé.

10. L'intermédiaire formé à l'étape (d) selon la revendication 8, qui est:

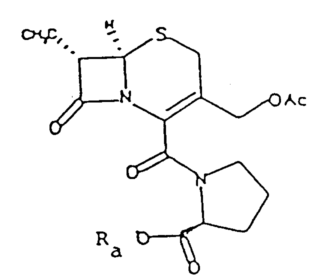

11. L'intermédiaire formé à l'étape (e) selon la revendication 8, qui est:

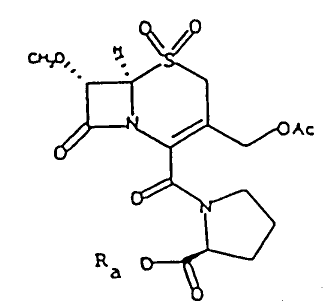

12. Une composition pharmaceutique pour traiter des états provoqués par l'élastase chez une espèce animale, comportant un support pharmaceutiquement non-toxique et une quantité efficace d'un composé des formules structurelles: 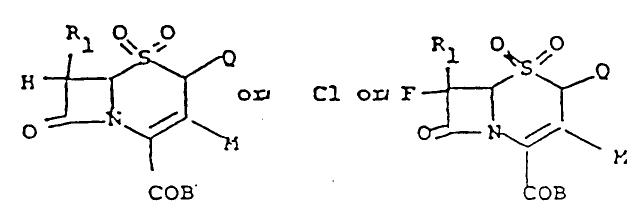 dans laquelle M, R₁, B et Q sont définis comme dans la revendication 1.

13. La composition de la revendication 12, dans laquelle le composé actif est le 3-acétyloxyméthyl-7 α-méthoxy-8-oxo-5-thia-1-azabicyclo[4.2.0]oct-2-ène-2-(2-(S)-carboxy-pyrrolidinecarboxamide)-5,5-dioxyde.

14. L'utilisation d'un composé des formules structurelles: 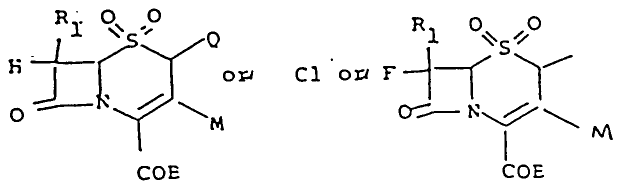 dans laquelle M, R₁, B et Q sont définis dans la revendication 1 pour la préparation d'un médicament utilisable pour le traitement ou la gestion des maladies relayées par l'élastase.

## Revendications (Revendications pour l'(les) Etat(s) contractant(s) suivant(s): ES, GR)

1. Un procédé pour la préparation d'un composé de la formule structurelle 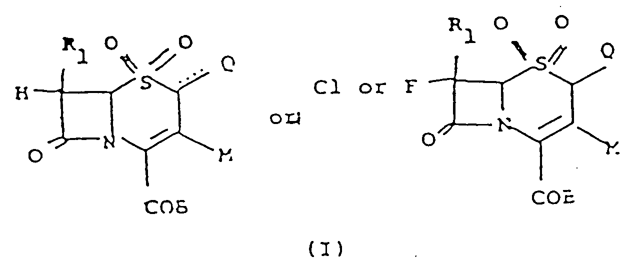 où M est:
(1) trifluorométhyle;
(2) chlore ou fluor;
(3) -COOH;
(4) -CHO; ou
(5) -CH₂A où A représente
(a) de l'hydrogène;
(b) de l'halogène;
(c) ZR₅ où Z est de l'oxygène et R₅ est de l'hydrogène ou un alkyle en C₁ à C₃, à chaîne droite ou ramifiée,
d) un acyloxy où l'acyle est un alcanoyle en C₂ à C₆, un succinoyle, ou bien un carbamoyle ou thiocarbamoyle et des dérivés N-alkyle ou N,N-dialkyle de ceux-ci dans lesquels les groupes alkyle renferment de 1 à 10 atomes de carbone et peuvent être en outre substitués par un amino ou un carboxyle;
(f) un acylthio dans lequel le groupe acyle est tel que défini au point (d);
(g) 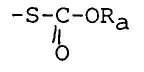 où Rₐ représente un acyle en C₁ à C₆, un phényle ou CH₂COOH;
(h) un radical amino, acétamido, carbamoylamino, N,N-diméthyl-amino, N-(2-chloroéthyl)amino, 5-cyanotriazolyle, 4-méthoxy carbonyl-triazol-1-yle;
(i) Rₐ-SO- où Rₐ est un alkyle en C₁ à C₁₆ ou un aryle en C₆ à C₁₀,
(j) Rₐ-SO₂- où Rₐ est défini au point (i);
(k) un substituant choisi dans le groupe constitué de 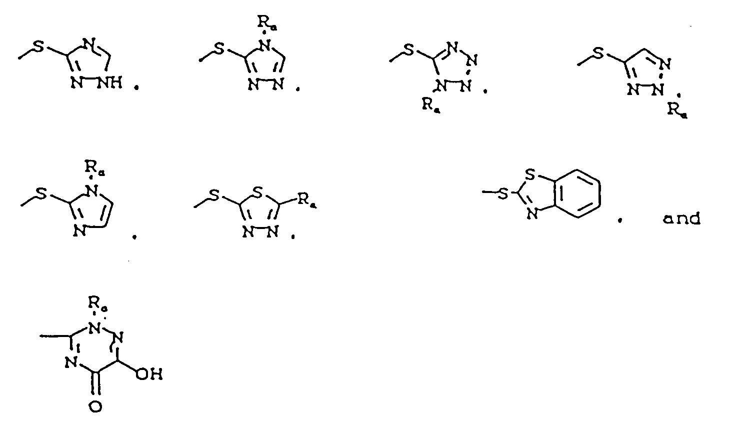 où Rₐ est un alkyle en C₁ à C₁₆, un aryle en C₆ à C₁₀ ou CH₂CO₂H;
(6) -CH=CH-Rₐ où Rₐ est tel que défini au point (i);
R₁ est
(a) de l'hydrogène;
(b) un radical hydroxy;
(c) un mercapto;
(d) OR¹₁ où R¹₁ est un groupe hydrocarbyle choisi dans un groupe constitué par un alkyle en C₁ à C₆, à chaîne droite ou ramifiée, un alcényle en C₃ à C₆, un alcynyle en C₃ à C₆, du phényle et du benzyle, ces groupes hydrocarbyle peuvent être non substitués ou substitués par un ou plus d'un radical qui est un hydroxy, de l'halogène, un nitro, un radical amino ou carboxy;
(e) -SR¹₁ où R¹₁ est tel que défini auparavant;
(f) un groupe hydrocarbyle choisi dans un groupe constitué de
(1) un alkyle en C₁ à C₆, à chaîne droite ou ramifiée;
(2) un alcényle en C₂ à C₆ à chaîne droite ou ramifiée;
(3) un alcynyle en C₂ à C₆ à chaîne droite ou ramifiée;
(4) du benzyle ou du phénéthyle;
(5) du cyclopropyle ou du cyclopentyle;
(6) du phényle;
(7) -CF₃;
les groupes ci-dessus (1) à (6) peuvent être non substitués ou bien substitués par un ou plus d'un radical choisi dans un groupe constitué par les radicaux halo, hydroxy, amino, nitro, sulfonyle, sulfamoyle, acyloxy, carbamoyloxy, carboxy ou carboxamido;
(g) du cyano;
(h) 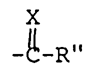 où X est de l'oxygène ou du soufre et R'' est
(1) de l'hydrogène;
(2) de l'hydroxy;
(3) un radical mercapto;
(4) un radical amine;
(5) un radical N-alkyle ou N,N-dialkylamine;
(6) un alkyle en C₁ à C₆;
(7) du benzyloxy;
(8) du phénoxy;
(9) un alkylthio en C₁ à C₆; ou
(10) un radical phénylthio;
(i) un radical halo;
(j) 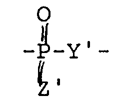 où Y' et Z' sont indépendamment OH, ou un radical -O-alkyle en C₁ à C₆;
(k) 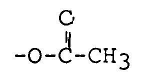
(l) -SO₂NH₂;
(m) -SO₂R₂ où R₂ est un radical alkyle en C₁ à C₆, halo-alkyle en C₁ à C₆, phényle ou benzyle;
(n) -SO₂NR₃R₄ où R₃ et R₄ représentent de façon indépendante, H ou un alkyle en C₁ à C₆;
B est un groupe hétérocyclique choisi dans le groupe comportant 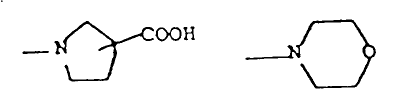 Q est
(1) de l'hydrogène;
(2) un alkyle en C₁ à C₆;
(3) un radical halo-alkyle en C₁ à C₆;
(4) un radical hydroxy-alkyle en C₁ à C₆;
(5) un radical méthylène, alkyl (en C₁ à C₆)méthylène, phényl-méthylène, phénylthio-méthylène, phénylsulfinyl-méthylène, ou phénylsulfonyl-méthylène;
(6) un alcoxy (en C₁ à C₆)-alkyl(en C₁ à C₆);
(7) du benzyle;
(8) un radical phénylthio-alkyle en C₁ à C₆, phénylsulfinyl-alkyle en C₁ à C₆ ou phénylsulfonyl-alkyle en C₁ à C₆;
(9) un radical phénoxy-alkyle en C₁ à C₆; ou
(10) un radical phénylamino-alkyle en C₁ à C₆.

2. Un procédé pour préparer un composé tel que défini dans la revendication 1
(a) l'estérification d'un composé de formule 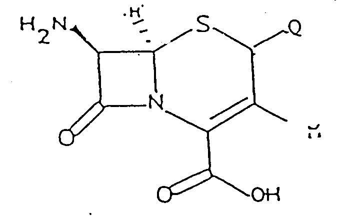 afin d'obtenir un ester de formule: 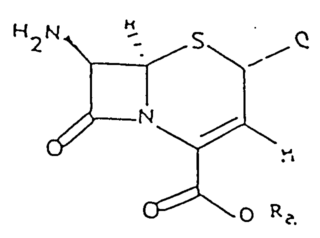 dans laquelle Q et M sont tels que définis auparavant; et Rₐ est un groupe protecteur;
(b) la diazotation de l'ester de l'étape (a) avec du NaNO₂ ou d'autres réactifs de diazotation et la conversion du dérivé diazoté obtenu avec du méthanol et de l'acétate de rhodium dimère pour former un ester de la formule 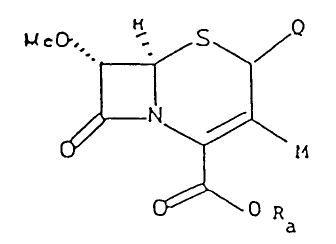 dans laquelle Rₐ est un groupe protecteur comportant un radical alkyle en C₁ à C₆, benzyle et benzylhydryle;
(c) la conversion de l'ester formé à l'étape (b) en un acide libre de formule: 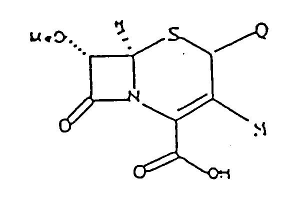
(d) la réaction de l'acide libre formé à l'étape (c) avec un composé hétérocyclique de formule HB en présence de N-hydroxysuccinimide, de DCC et de triéthylamine afin de former l'amide de formule 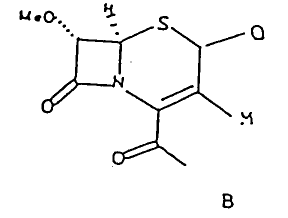 dans laquelle B est défini auparavant; ou, en variante l'estérification de l'acide libre de l'étape (d) pour former un ester de la formule: 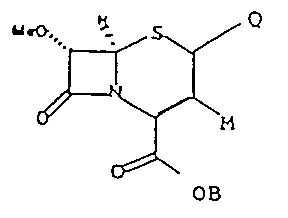 dans laquelle B est tel que défini auparavant; et
(e) l'oxydation du produit de l'étape (d) avec de l'acide m-chloroperbenzoïque ou d'autres agents d'oxydation appropriés afin de former une sulfone de formule 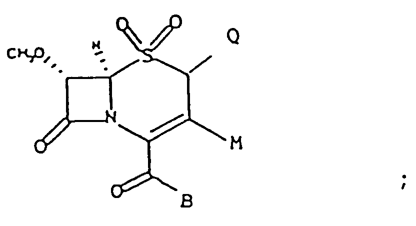 et, le cas échéant
(f) la conversion de la sulfone de l'étape (e) en un composé de formule (I) par déblocage du groupe protecteur.

3. Le procédé de la revendication 2, dans lequel le composé de la formule 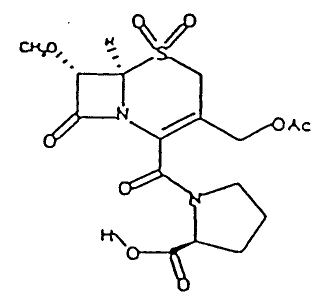 est préparé

4. Un procédé tel que revendiqué dans les revendications 1 ou 2, dans lequel est préparé un composé de la formule: 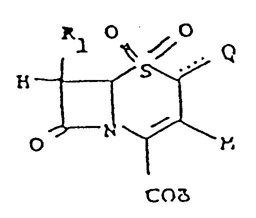 dans laquelle M est:
(1) du trifluorométhyle;
(2) un radical chloro ou fluoro; ou
(3) -CH₂A où A représente:
(a) un alcanoyloxy de 2 à 6 atomes de carbone;
(b) un alcoxy de 1 à 3 atomes de carbone;
(c) un radical halo;
(d) de l'hydrogène;
(e) de l'hydroxy;
(f) un radical mercapto ou mercapto substitué par un groupe aryle de 2 à 6 atomes de carbone;
(g) du carbamoyloxy;
(h) -SO-CH₃ ou -SO-C₆H₅
(i) -SO₂-CH₃ ou -SO₂-C₆H₅;
R₁ est :
(1) un alkyle en C₁ à C₆;
(2) de l'hydroxy
(3) OR: où R¹ est
(a) un radical alkyle en C₁ à C₆, alcényle en C₃ à C₆ ou alcynyle en C₃ à C₆;
(b) -C₆H₅;
(c) CH₂CH₂C₆H₅ ou
(d) -CO-R¹¹ où R¹¹ est de l'hydrogène, un radical alkyle en C₁ à C₆, phényle, benzyle ou alkylamino en C₁ à C₆;
(4) un radical fluoro ou chloro;
B est tel que défini dans la revendication 1 et Q est
(1) de l'hydrogène;
(2) un radical méthyle, éthyle ou bien i- ou n-propyle;
(3) du méthylène ou
(4) un radical phénylthiométhyle ou phénylsulfonylméthyle.

5. Un procédé tel que revendiqué dans la revendication 4, dans lequel Q est de l'hydrogène.

6. Un procédé tel que revendiqué dans la revendication 4 ou 5, dans lequel R₁ est du méthoxy.

7. Un procédé tel que revendiqué dans l'une quelconque des revendications 4 à 6, dans lequel M est CH₂A, où A est un alcanoyloxy.
